(19) Europäisches Patentamt
European Patent Office
Office européen des brevets

(11) Publication number: 0 204 344 B1

(12) # EUROPEAN PATENT SPECIFICATION

(45) Date of publication of patent specification: 20.09.89

(51) Int. Cl.[4]: C 07 H 3/04, C 07 H 15/04

(21) Application number: 86107723.8

(22) Date of filing: 06.06.86

(54) **Sialosylcerebrosides and a preparation method thereof.**

(30) Priority: 07.06.85 JP 123951/85

(43) Date of publication of application:
10.12.86 Bulletin 86/50

(45) Publication of the grant of the patent:
20.09.89 Bulletin 89/38

(84) Designated Contracting States:
AT BE CH DE FR GB IT LI LU NL SE

(56) References cited:
BIOCHEMISTRY, vol. 21, no. 6, 16th March 1982, pages 1260-1271, American Chemical Society, US; L.O. SILLERUD et al.: "Assignment of the carbon-13 nuclear magnetic resonance spectra of gangliosides GM4, GM3, GM2, GM1, GD1a, GD1b, and GT1b"
CHEMICAL ABSTRACTS, vol. 68, no. 3, 15th January 1968, pages 900-901, abstract no. 9505e, Columbus, Ohio, US; E. KLENK et al.: "Two components of a mixture of brain gangliosides", & HOPPESEYLER'S Z. PHYSIOL. CHEM. 348 (10), 1261-7
Carbohydrate Research, vol.135(1985), pp. C5-C9

(73) Proprietor: RIKAGAKU KENKYUSHO
2-1 Hirosawa
Wako-shi Saitama-ken (JP)
(73) Proprietor: MECT CORPORATION
1-1, Nishishinjuku 2-Chome
Shinjuku-ku Tokyo 163 (JP)

(72) Inventor: Ogawa, Tomoya
No. 3-6-6-3-101, Kitamachi Kichijyouji
Musashino-shi Tokyo (JP)
Inventor: Sugimoto, Mamoru
No. 4-6-32, Sakae
Niiza-shi Saitama-ken (JP)
Inventor: Numata, Masaaki
No. 1758, Ohaza Yamada
Kawagoe-shi Saitama-ken (JP)
Inventor: Shitori, Yoshiyasu
No. 12-6-1307, Nishishinjyuku 6-chome
Shinjyuku-ku
Tokyo (JP)
Inventor: Ito, Masayoshi
No. 1-27-22-303, Fujimidai
Kunitachi-shi Tokyo (JP)

(74) Representative: Vossius & Partner
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 204 344 B1

Courier Press, Leamington Spa, England.

## Description

This invention relates to sialosylcerebrosides and to processes for their preparation.

Glycolipids found in mammal cells are glycosides between ceramides, which are sphingosines (long chain amino alcohols) to which aliphatic carboxylic acids have been attached through an amide linkage, and one or more sugars such as glucose, galactose, N-acetyl glucosamine, N-acetyl galactosamine, fucose, sialic acid, etc. Among these glycosides, those containing sialic acid are called gangliosides.

Gangliosides exist mainly in the outer molecular layer of the double molecule layers of the cell membrane of mammals. Recent studies show that gangliosides play an important role in reception and recognition of, and response to, information in the cell, receptor mechanism, differentiation, cell propagation, malignant cell transformation, cell behavior, etc.

Among these compounds, sialosylcerebroside $GM_4$ is contained in large amounts in the gray substance of the human brain. Sialosylcerebroside $GM_4$ is considered a distinctive component of the myelin membrane. These acidic glycolipids are found in mouse erythrocytes, rat kidneys and egg yolk.

Ganglioside $GM_4$, prepared from chicken brains has been reported to have the structure NeuAcα2→3Galβ1→1Cer (Biochemistry Vol. 21 (1982), pages 1260—1271).

However, the function of ganglioside glycolipids as a cell membrane component has not been studied sufficiently and it is very difficult to isolate and purify sialosylcerebroside $GM_4$ from natural sources. Therefore a synthesis of such sialosylcerebrosides containing sialosylcerebroside $GM_4$ analogues is necessary for the investigation of the function of ganglioside glycolipids as a cell membrane component.

An object of this invention is to provide novel sialosylcerebrosides and methods for their preparation, and a method for the preparation of sialosylcerebroside $GM_4$.

The novel sialosylcerebrosides of the present invention have the general formula I

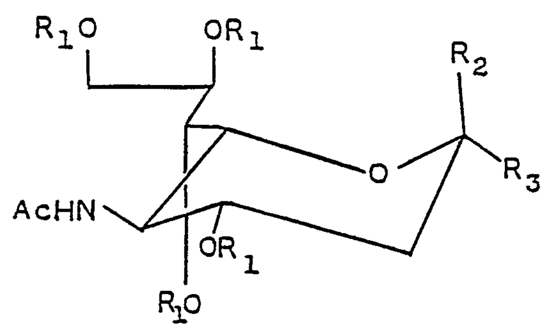

(I)

wherein $R_1$ is hydrogen or an acetyl group, $R_2$ is $-COOR_4$, wherein $R_4$ is hydrogen, sodium or a methyl group, and $R_3$ is the group

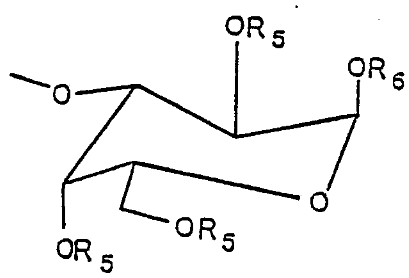

wherein $R_5$ is hydrogen or an acetyl group, $R_6$ is $-C(CCl_3){=}NH$ or the group

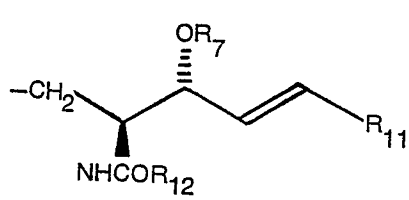

wherein $R_7$ is hydrogen or a benzoyl group, $R_{11}$ is a saturated aliphatic hydrocarbon group having 10 to 16

carbon atoms and $R_{12}$ is a saturated aliphatic hydrocarbon group having 15 to 25 carbon atoms, or $R_2$ is the group

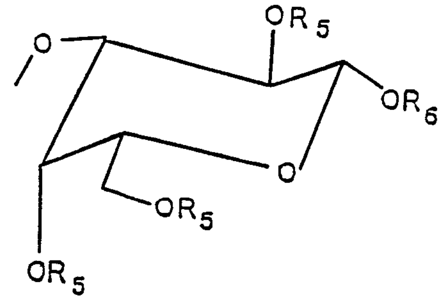

wherein $R_5$ and $R_6$ have the same meaning as defined above and $R_3$ is $-COOR_4$, wherein $R_4$ has the same meaning as defined above.

The invention will now be explained in detail.

(a) Synthesis of the ceramide moiety

The ceramide moiety of gangliosides can be prepared by the method as shown in Scheme 1b. Compound (30) can be prepared by the method as shown in Scheme 1a (see Japanese Patent Application No. 59—44913 (Japanese Patent Public Disclosure No. 60—190745)).

The compound ② can be obtained by refluxing overnight a solution of a corresponding alkyl halide such as 1-bromotetradecane and triphenylphosphine in a solvent such as xylene.

1,2-O-isopropylidene-α-D-xylo-pentodialdo-1,4-furanose ① is reacted with the compound ② in the presence of BuLi in a solvent such as THF or hexane to prepare the 4-alkylvinyl derivative ③. The reaction temperature and time are suitably −15°C to 25°C and 0.5 to 24 hours, respectively.

The compound ③ is treated with methanesulfonyl chloride (MsCl) in dry pyridine to produce the 3-methanesulfonyl derivative ④. The reaction is suitably carried out at 0°C to 25°C for 2 to 24 hours.

Treatment of the compound ④ in acetic acid/water removes the isopropylidene group to yield the diol ⑤. The reaction is suitably carried out at 70 to 90°C for 0.5 to 5 hours.

The compound ⑤ is treated with an oxidizing agent such as sodium metaperiodate in a solvent such as ethanol to cleave the glycol and then treated with a reducing agent such as sodium borohydride to obtain the diol compound ⑥. The oxidation is carried out at 0°C to 25°C for 0.5 to 24 hours and the reduction at 0°C to 10°C for 0.5 to 2 hours.

The compound ⑥ is reacted with an alkyl vinyl ether such as ethyl vinyl ether in a solvent such as dichloromethane in the presence of a catalyst such as pyridinium p-toluenesulfonate to obtain the di-alkyl vinyl ether ⑦. This reaction is suitably carried out at 0°C to 30°C for 0.5 to 24 hours.

The compound ⑦ is treated with an azide such as sodium azide in an aprotic solvent such as DMF (dimethylformamide) to obtain the compound ⑧. This reaction is suitably carried out at 70°C to 120°C for 15 hours to 6 days.

The azide ⑧ is treated with a reducing agent such as sodium borohydride or Lindlar catalyst/$H_2$ in a solvent such as ethanol or isopropanol to obtain the amine ⑨. This reaction is suitably carried out at reflux temperature for 1 to 6 days when sodium borohydride is used and at 0°C to 30°C for 2 to 24 hours at a hydrogen pressure of 1 to 4 at when Lindlar catalyst/$H_2$ is used.

The amine ⑨ is reacted with an acyl halide in the presence of a basic compound such as pyridine or dimethylaminopyridine to obtain the amide ⑩ or ⑪. This reaction is suitably carried out at 0°C to 30°C for 0.5 to 24 hours. Alternatively, the amine ⑨ is dissolved in dichloromethane or the like and reacted with a corresponding aliphatic carboxylic acid in the presence of 2-chloro-1-methylpyridinium iodide, tri-n-butyl amine, etc. to obtain the amide ⑩ or ⑪. This reaction is carried out at reflux temperature for 0.5 to 13 hours in an inert gas atmosphere such as argon.

The amide ⑩ or ⑪ is then treated with e.g. pyridinium p-toluenesulfonate or Amberlyst® 15 in a solvent such as methanol, dichloromethane to remove the protective groups. Thus the ceramide ⑫ or (31) is obtained; see Scheme 1a.

Note: EE = ethoxyethyl $-CH(CH_3)-O-C_2H_5$

Scheme 1a

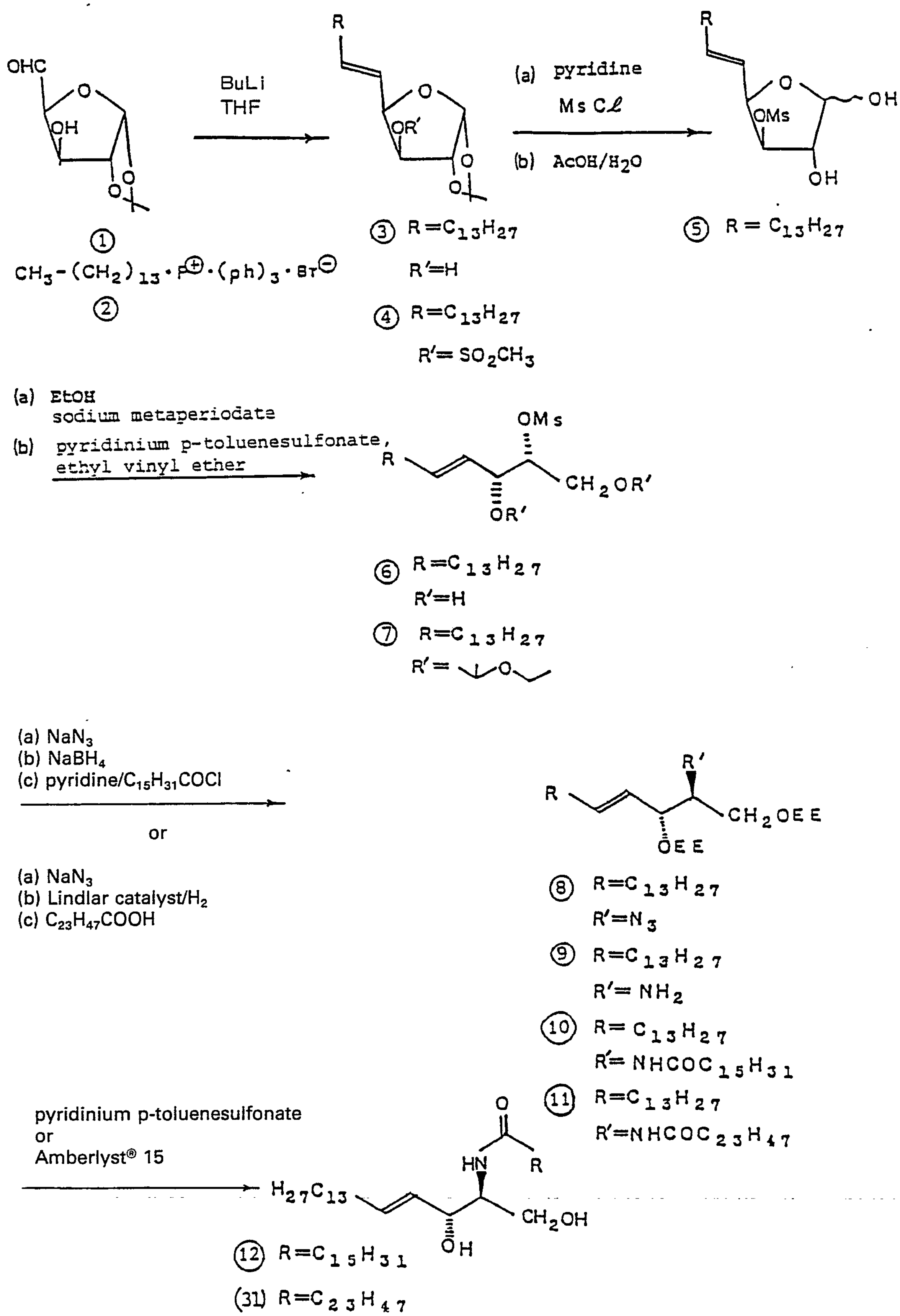


(1)

$CH_3-(CH_2)_{13}\cdot P^{\oplus}\cdot(ph)_3\cdot Br^{\ominus}$

(2)

(3) $R=C_{13}H_{27}$
$R'=H$

(4) $R=C_{13}H_{27}$
$R'=SO_2CH_3$

(5) $R=C_{13}H_{27}$

(6) $R=C_{13}H_{27}$
$R'=H$

(7) $R=C_{13}H_{27}$

(8) $R=C_{13}H_{27}$
$R'=N_3$

(9) $R=C_{13}H_{27}$
$R'=NH_2$

(10) $R=C_{13}H_{27}$
$R'=NHCOC_{15}H_{31}$

(11) $R=C_{13}H_{27}$
$R'=NHCOC_{23}H_{47}$

(12) $R=C_{15}H_{31}$

(31) $R=C_{23}H_{47}$

The compound (31) thus obtained is treated with trityl chloride (TrCl) in pyridine to obtain the trityl derivative (32) which is then treated with benzoyl chloride (BzCl) and dimethylaminopyridine to obtain the trityl-benzoyl derivative (33) which is then treated with p-toluenesulfonic acid (p-TsOH) to remove the trityl group. Benzoyl ceramide (34) is obtained. The compound (34) can be obtained without isolation of the compounds (32) and (33); see Scheme 1b.

Scheme 1 b

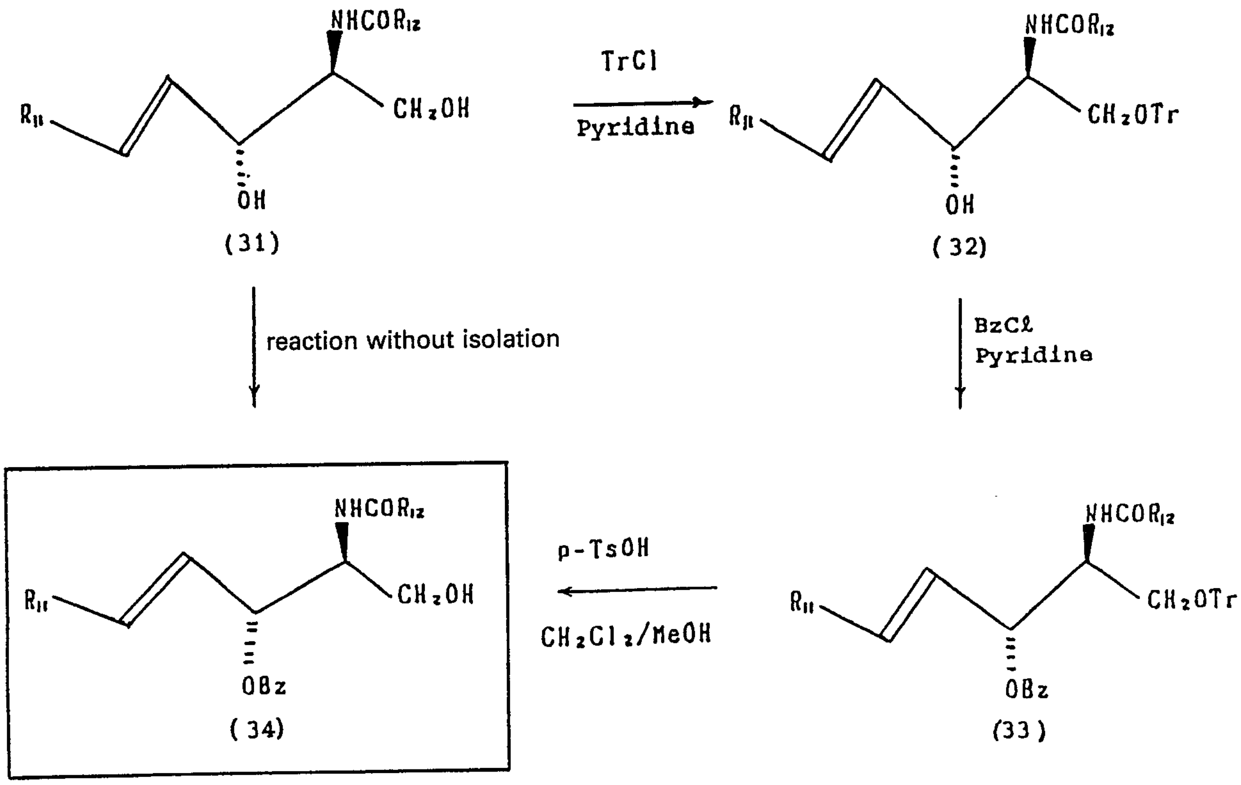


$R_{11}$: $C_{13}H_{27}$, $R_{12}$: $C_{23}H_{47}$, Tr: Trityl group, Bz: Benzoyl group

(b) Synthesis of the sialic acid derivatives (10) and (20)

The sialic acids (10) and (20) which are used to synthesize the sialosylcerebrosides of the present invention can be obtained as described in Carbohydrate Research, vol. 135 (1985), pages C5 to C9. This synthesis involves the reaction of compound (D) obtained from benzylgalactoside (A) as shown in Scheme 2 with N-acetyl neuramic acid acetate methyl ester (compound (E)) synthesized according to the method of R. Kühn et al., Chem. Ber., Vol. 99 (1966), 611—617.

Benzylgalactoside (A) is suspended in acetone and reacted with 2,2-dimethoxypropane in the presence of p-toluenesulfonic acid (p-TsOH) to obtain the 3,4-O-isopropylidene derivative (B). The derivative (B) is reacted with benzylbromide (BmBr) in DMF in the presence of NaH to obtain the tribenzyl derivative (C). The derivative (C) is treated with an 80% acetic acid solution to remove the isopropylidene group. Compound (D) is obtained. The reaction of compound (D) with (E) may be carried out in a solvent such as dichloromethane or 1,2-dichloroethane in the presence of a glycosidation catalyst such as $Hg(CN)_2$, $HgBr_2$, a molecular sieve (MS), $Ag_2CO_3$, $AgClO_4$, $AgOSO_2CF_3$ or $(CH_3)_3COSO_2CF_3$ at −20 to 150°C for 1 to 120 hours; see Scheme 2.

Scheme 2

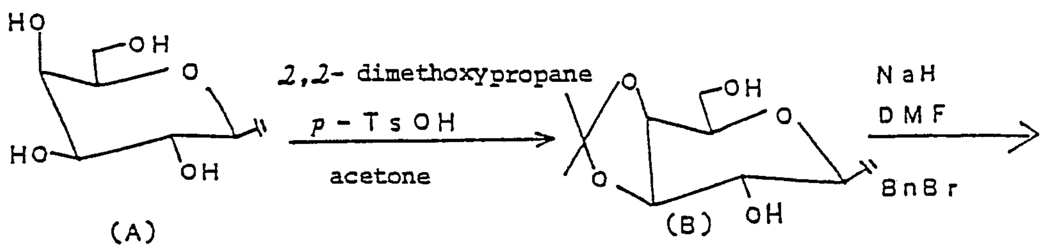


(A) (B)

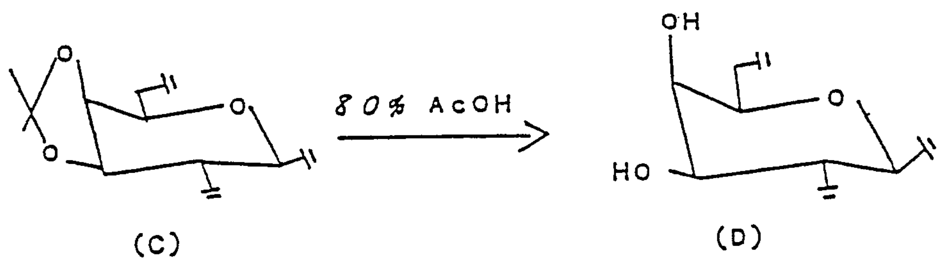


(C) (D)

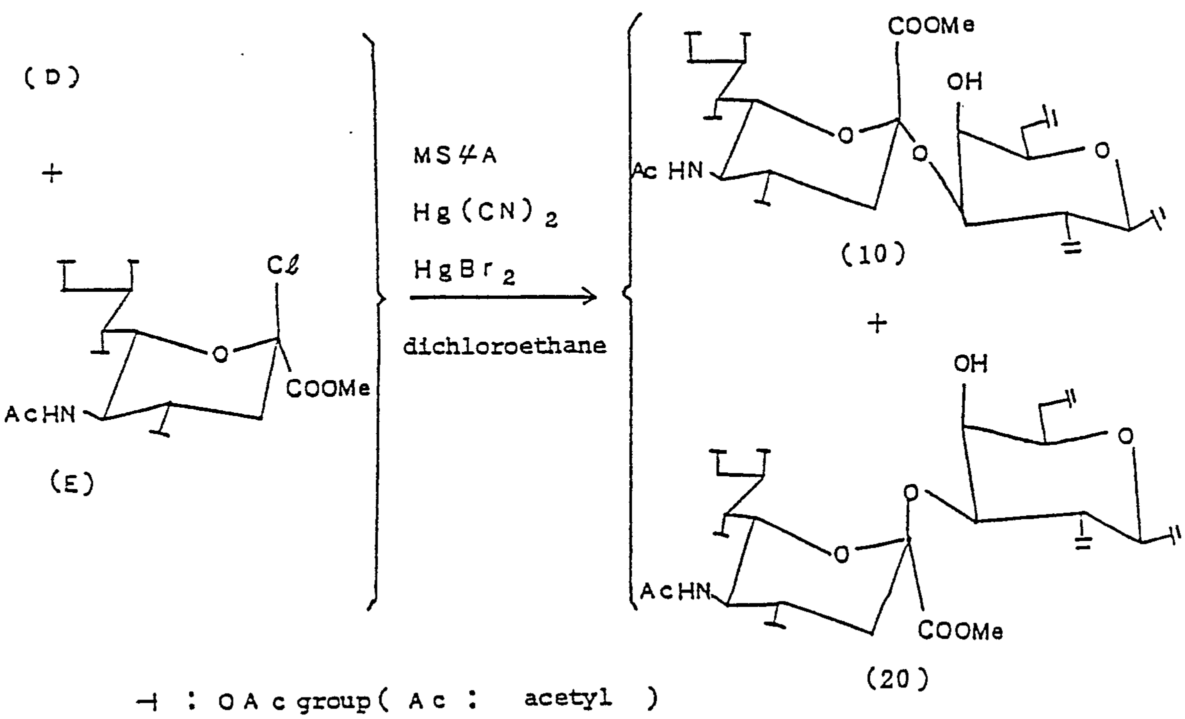


(D) + (E) → (10) + (20)

⊣ : OAc group ( Ac : acetyl )

⊣ı : OBn group ( Bn : benzyl )

(c) Synthesis of sialosylcerebrosides

The sialosylcerebrosides of the present invention can be obtained from the sialic acid derivatives (10) and (20) as shown in schemes 3 and 4.

Compound (11) is obtained by acetylation of the sialic acid derivative (10) with acetic anhydride and pyhridine. Compound (11) is treated with Pd—C and MeOH to remove the benzyl group and then the product obtained is acetylated by treating it with acetic anhydride and pyridine to obtain compound (12). Then compound (12) is treated with $NH_2NH_2AcOH$ and DMF to obtain the deacetylated compound (13).

Compound (13) is reacted with trichloroacetonitrile in a solvent such as methylene chloride in the presence of NaH to obtain compound (14). This reaction may be carried out, for example, at a temperature of −10 to 30°C, preferably under ice cooling for 1 to 5 hours and under agitation.

Compound (14) is reacted with the benzoyl derivative of ceramide (Bz ceremide) (34), for example, in a solvent such as chloroform in the presence of MS4A or $BF_4 \cdot EtO_2$ to obtain compounds (15) and (16). This reaction may be carried out at a temperature of −40 to 40°C for 0.5 to 2 hours, then at a temperature of 0 to 40°C for 2 to 24 hours and under agitation. Compounds (15) and (16) can be separated by a method such as column chromatography on silicagel.

Compounds (17) and (18) can be obtained by, for example, treating compounds (15) and (16) respectively with NaOMe in a solvent such as MeOH/THF or the like and if necessary treating the resultant product with Amberlite® IRC—50S50 or the like. The treatment with NaOMe is preferably carried out at a temperature of 0 to 30°C for 0.5 to 6 hours.

On the other hand, the sialic acid derivative (20) is acetylated with dimethoxyaminopyridine in a mixture of pyridine and acetic anhydride to obtain compound (21). Compound (21) is treated with Pd—C and MeOH to remove the benzyl group. Compound (22) is obtained. Compound (22) is acetylated by treating it with 4-dimethoxyaminopyridine in a mixture of pyridine and acetic anhydride. Compound (23) is obtained. Compound (23) is treated with $NH_2NH_2AcOH$ in DMF to remove the acetyl group. Compound (24) is obtained.

Compound (24) is reacted with trichloroacetone, for example, in a solvent such as dichloroethane or the like in the presence of DBU to obtain compound (25). This reaction may be carried out at a temperature of −10 to 30°C for 30 minutes to 6 hours.

Compound (25) is reacted with the benzoyl derivative of ceramide (IV), for example, in a solvent such as chloroform or the like in the presence of MS 4A and $BF_3 \cdot Et_2O$ to obtain compound (26). This reaction may be carried out under agitation at a temperature of −10 to 40, preferably 0 to 20°C, for 2 to 5 hours, followed by additional agitation at a temperature of 20 to 40°C for 1 to 24 hours; see Scheme 3 and 4.

Scheme 3

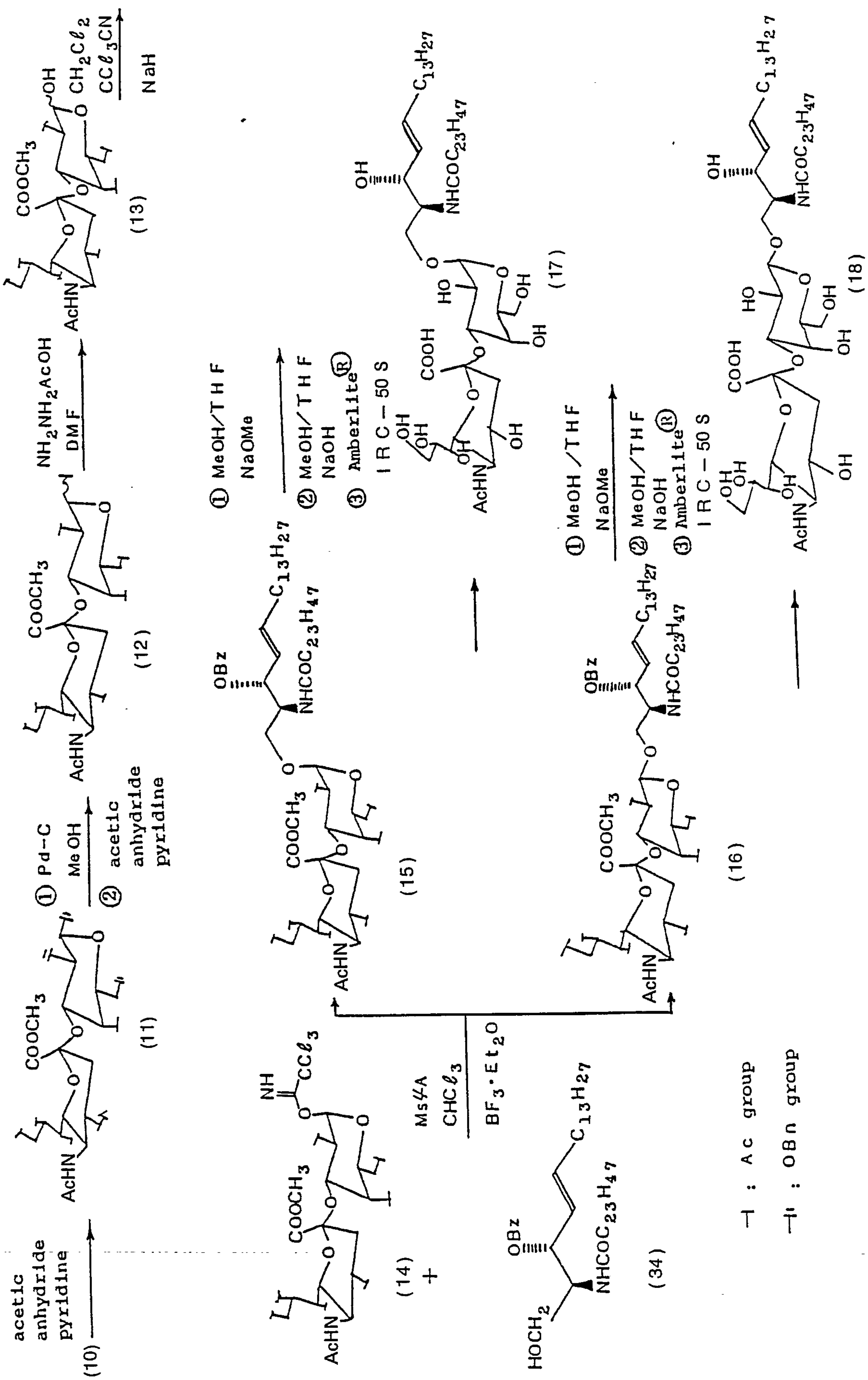
(10)
acetic anhydride pyridine
(11)
① Pd-C MeOH
② acetic anhydride pyridine
(12)
$NH_2NH_2AcOH$ DMF
(13)
$CH_2Cl_2$ $CCl_3CN$ NaH
(14)
+
(34)
Ms4A $CHCl_3$ $BF_3 \cdot Et_2O$
(15)
(16)
① MeOH/THF NaOMe
② MeOH/THF NaOH
③ Amberlite® IRC-50 S
(17)
① MeOH/THF NaOMe
② MeOH/THF NaOH
③ Amberlite® IRC-50 S
(18)
⊣ : Ac group
⊣⁝ : OBn group

Scheme 4

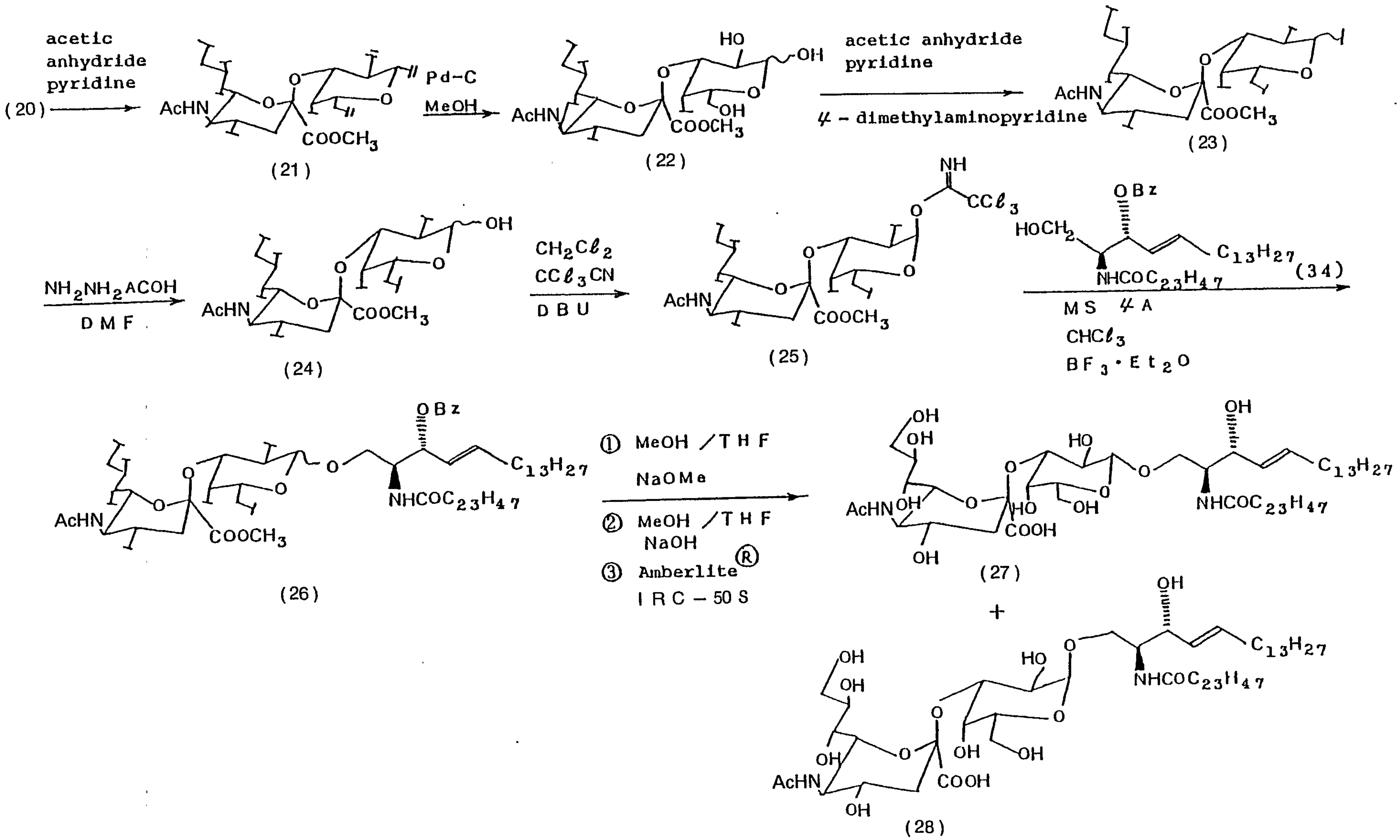

(20)
acetic anhydride pyridine
(21)
Pd-C MeOH
(22)
acetic anhydride pyridine
4 - dimethylaminopyridine
(23)
NH2NH2ACOH DMF
(24)
CH2Cℓ2 CCℓ3CN DBU
(25)
(34)
MS 4A
CHCℓ3
BF3・Et2O
(26)
① MeOH /THF NaOMe
② MeOH /THF NaOH
③ Amberlite® IRC-50S
(27)
+
(28)

Compound (26) is treated with $NaOCH_3$ in a solvent such as a mixture of THF and MeOH to remove the benzoyl group. Compound (27) is obtained. This treatment may be carried out under agitation of the solution containing compound (26) at a temperature of 0 to 40°C for 1 to 24 hours. The treatment can include an additional treatment with $H_2O$ in a mixture of THF and MeOH and neutralization of the solution with Amberlite® IRC—50S.

Compounds (14), (15), (16), (17), (25), (26) and (27) obtained by the method of the present invention are novel compounds.

The novel compounds of the present invention are useful as tumor markers, differentiation markers for cells having inducing ability of differentiation and synthesis intermediates thereof.

The present invention will be illustrated by the following examples.

Reference Example 1

From compound (11) to compound (12)

658 mg (0.68 mmol) of compound (11) were dissolved in 25 ml of methanol and reduced catalytically at room temperature for 24 hours using 320 mg of 10% Pd—C. After the reaction, the reaction mixture was filtered to remove Pd—C.

The filtrate was concentrated under reduced pressure. The residue obtained (463 mg, 98%, Rf 0.72, $BuOH:EtOH:H_2O = 4:2:2$) was dissolved in a mixture of 2 ml of acetic anhydride and 2 ml of pyridine, agitated at room temperature for 24 hours and concentrated under reduced pressure. The residue was purified by chromatography on silicagel (Wakogel® C—300, 35 g, toluene:ethyl acetate = 1:4). 545 mg of compound (12) were obtained (yield: 85%).

$[\alpha]_D^{22}$ +7.60 (c = 0.96, $CHCl_3$).
$R_f$ = 0.38, EtOAc, HPTLC.
Elemental analysis
Calculated C, 49.69, H, 5.77, N, 1.70
Found C, 49.73, H, 5.78, N, 1.57
NMR (400 MHz ppm, $CDCl_3$, TMS)
1.71, 1H, t, J = 12.5, H-3bax,
2.60, 1, H, m .H-3beq 1.83—2.23
27 H, $CH_2CO$, 3.86, S, —$OCH_3$
α-anomer, 3.85 S, —$OCH_3$ β-anomer,
5.38 d, J = 8.3 H-1aβ 6.29
H-1aα d, J = 3.91

Reference Example 2

From compound (12) to compound (13)

452 mg (0.55 mmol) of compound (12) were dissolved in 1.0 ml of DMF and heated to 50°C. Then 56 mg of $NH_2NH_2AcOH$ were added and the mixture was agitated for 5 minutes. After the reaction mixture was cooled, the mixture was diluted with ethyl acetate and washed with water. The ethyl acetate phase was dried over $MgSO_4$ and concentrated to obtain 414 mg of compound (13) (yield: 97%).

$R_f$ = 0.32 EtOAc, HPTLC.

Example 1

From compound (13) to compound (14)

156 mg of compound (13) were dissolved in 1.0 ml of methylene chloride. Then 116 mg (0.80 mmol) of trichloroacetonitrile were added. To the mixture obtained were added 9.0 mg of NaH (60% dispersion in oil) under agitation while cooling with an ice bath. The mixture was agitated for 3 additional hours. The reaction mixture was concentrated under reduced pressure and purified by chromatogarphy on silicagel (Wakogel® C—300, 10 g, EtOAc). 84 mg of compound (14) (yield: 45%) were obtained.

$R_f$ = 0.37, EtOAc.

Example 2

From compound (14) to compounds (15) and (16)

62 mg (0.067 mmol) of compound (14) and 51 mg (0.067 mmol) Bz-ceramide (34) prepared by the method as shown in Scheme 1a and 1b were dissolved in 2 ml of chloroform. To the chloroform solution were added 1 g of activated molecular sieve AW—300 and 10 µl (0.08 mmol) of $BF_3\cdot Et_2O$ under agitation while cooling with an ice bath. The reaction mixture was agitated at the same temperature for 1 hour and agitated at room temperature for 24 hours. Then the reaction mixture was diluted with chloroform, filtrated with Celite® and concentrated under reduced pressure. The residue was purified by chromatography on silicagel (Wakogel® C—300, 20 g, toluene:ethyl acetate = 1:2). 10.4 mg of compound (15) (yield: 10.2%) and 28 mg of compound (16) were obtained (yield: 27.5%).

Compound (15)

$R_f$ = 0.41 (toluene:ethyl acetate = 1:2)

Elemental analysis

Calculated C, 64.18, H, 8.64, N, 1.85

Found C, 64.14, H, 8.71, N, 1.87

$[\alpha]_D^{20}$ −17.2 (c = 0.92, $CHCl_3$).

NMR (400 MHz ppm, $CDCl_3$, TMS)
0.876 $CH_3$ t J = 5.9, 0.879,
$CH_3$t J = 5.4, 1.252 $CH_2$ 1.917—
2.165 $CH_3CO$, 3.830 $CH_3O$—, S

Compound (16)

$R_f$ = 0.25 (toluene:ethyl acetate = 1:2)

$[\alpha]_D^{21}$ −0.37 (c = 1.40, $CHCl_3$)

Elemental analysis $C_{81}H_{130}N_2O_{24}$ + $\frac{1}{2}C_6H_5CH_3$

Calculated C, 64.98, H, 8.64, N, 1.79

Found C, 65.07, H, 8.77, N, 2.05

## Example 3

From compound (15) to compound (17)

25 mg (0.0165 mmol) of compound (15) were dissolved in 2.0 ml of a mixture of THF and MeOH (THF:MeOH = 1:1). Then 70 µl of 2.14N $NaHCO_3$ aqueous solution were added. The mixture was agitated at room temperature for 3 hours. The reaction mixture was concentrated under reduced pressure. To the residue were added 1.0 ml of THF and 1.0 ml of water. The mixture was agitated at room temperature for 1 hour. The mixture obtained was neutralized with Amberlite® IRC—50S and the Amberlite® was removed by filtration. The filtrate was dried under reduced pressure. The residue was dissolved in 2.0 ml of a mixture of THF and MeOH (THF:MeOH = 1:1). The mixture was made basic with an aqueous NaOH solution and concentrated under reduced pressure. The residue was dissolved in a mixture of chloroform, methanol and water (60:30:4.6) and purified by chromatography on Sephadex® LH—20 (1 × 45 cm). 12 mg of compound (17) were obtained (yield: 66%).

$R_f$ = 0.34, chloroform:methanol:water = 60:30:4.6.

NMR (400 MHz d.b DMSO, 30°C, TMS)
5.54, H-5′, t.d. J = 6.0, 16.4; 5.55,
H-4′, d, d, J = 7.0, 15.6, 4.66, bs
H-1a eq, 4.22, H-3a, dd J = 4.0,
7.0, 3.98 H-4a d, J = 2.4, 3.90
H-3′ t J = 7.0 1.23, s $CH_2$ 0.85,
$CH_3$, t J = 6.4

## Example 4

From compound (16) to compound (18)

10 mg (0.066 mmol) of compound (16) were dissolved in 2 ml of a mixture of THF and MeOH (1:1). To the solution obtained were added 15 µl of a 1N $NaOCH_3$ solution and agitated overnight at room temperature. To the reaction mixture were added 10 µl of a 1N $NaOCH_3$ solution. The mixture was agitated at room temperature for 4 hours. Then the solvent was removed under reduced pressure. To the residue were added 2 ml of a mixture of THF and MeOH (1:1) and 0.5 ml of $H_2O$ and the mixture was agitated overnight at room temperature. The solvent was removed under reduced pressure. The residue was purified by chromatography. Sephadex® LH—20 ($CHCl_3$:MeOH:$H_2O$ = 60:30:4.6). 5 mg of compound (18) were obtained (yield: 68.5%).

$R_f$ = 0.386 ($CHCl_3$:MeOH:$H_2O$ = 60:30:4.6).

$[\alpha]_D^{23}$ −2.545 (c = 0.165, $CHCl_3$:MeOH = 2:1).

NMR (400 MHz d-b DMSO, 30°C, TMS)
5.52, H-5′, d t, J = 6.6, 15.1;
5.34, H-4′, d d, J = 15.4, 7.1;
4.07, H-1, aax J = 7.6
2.76, H-3beq, dd, J = 12.5, 4.9:

## Reference Example 3

From compound (20) to compound (21)

3 ml of pyridine and 3 ml of acetic anhydride were added to 150 mg (0.16 mmol) of compound (20) and the compound was dissolved therein. To the mixture obtained were added 50 mg of 4-dimethoxyaminopyridine. The mixture was agitated at room temperature for 2 days. The solvent was

removed to obtain a residue which was purified by chromatography on silicagel (Wakogel® C—300, 20 g, toluene:MeOH = 10:1) to obtain 180 mg of compound (21) (yield: 89%).

$R_f$ = 0.439 (toluene:MeOH = 10:1)

$[\alpha]_D^{19}$ −19.29 (c = 0.985, $CHCl_3$)

Reference Example 4

From compound (21) to compound (22)

1.1449 g of compound (21) were dissolved in 50 mg MeOH and reduced catalytically at room temperature for 4 hours in the presence of 600 mg of 10% Pd—C. The reaction mixture was filtered through Celite® and distilled under reduced pressure. 807 mg of compound (22) were obtained (yield: 97.9%).

$R_f$ = 0.64 (BuOH:EtOH:$H_2O$ = 4:2:1).

Reference Example 5

From compound (22) to compound (23)

121 mg (0.174 mmol) of compound (22) were dissolved in a mixture of 3 ml of pyridine and 3 ml of acetic anhydride. To the solution were added 20 mg of 4-dimethoxyaminopyridine. The mixture was agitated overnight. The reaction mixture was distilled under reduced pressure and the residue was purified by chromatography on silicagel (Wakogel® C—300, 20 g, toluene:MeOH = 10:1). 110.4 mg of compound (23) were obtained (yield: 72.2%).

$R_f$ = 0.259 (toluene:MeOH = 10:1).

$[\alpha]_D^{19}$ +26.63 (c = 0.995, $CHCl_3$).

Elemental analysis $C_{34}H_{47}O_{22}N_1 + \frac{1}{2}C_6H_5CH_3$

| | | | |
|---|---|---|---|
| Calculated | C, 51.90%, | H, 5.92%, | N, 1.61 |
| Found | C, 51.71%, | H, 5.91%, | N, 1.69 |

Reference Example 6

From compound (23) to compound (24)

96 mg (0.12 mmol) of compound (23) were dissolved in 1 ml of DMF. To the solution were added 12.2 mg of $H_2N \cdot NH_2 \cdot AcOH$. The mixture was agitated at 50°C for 5 minutes. The reaction mixture was diluted with ethyl acetate and washed with water. The mixture obtained was dried over magnesium sulfate and distilled under reduced pressure. The residue was purified by chromatography on silicagel (Wakogel® C—300, 25 g, acetone:carbon tetrachloride = 1:1). 69.5% of compound (24) were obtained (yield = 76.4%).

$R_f$ = 0.509 (acetone:carbon tetrachloride).

$[\alpha]_D^{19}$ +36.16 (c = 1.015, $CHCl_3$).

Example 5

From compound (24) to compound (25)

133 mg (0.17 mmol) of compound (24) were dissolved in 1 ml of dried dichloroethane. To the solution were added 0.358 ml of $Cl_3CCN$ and 12 µl (0.085 mmol) of DBU while cooling with an ice bath. The mixture was agitated for 3 additional hours. The reaction mixture was purified by chromatography on silicagel (Wakogel® C—300, 20 g, acetone:carbon tetrachloride = 1:2) to obtain 122 mg of compound (25) (yield: 77.4%). Note: DBU is 1,8-diazabicyclo[5.4.0]undec-7-ene.

$R_f$ = 0.393 (acetone:carbon tetrachloride = 1:2).

$[\alpha]_D^{19}$ +35.18 (c = 1.00, $CHCl_3$).

Example 6

From compound (25) to compound (26)

To 100 mg (0.108 mmol) of compound (25) and 83 mg (0.109 mmol) of Bz-ceramide dissolved in 3 ml of chloroform were added 1 g of molecular seive AW—300. To the mixture were added 15 µl (0.124 mmol) of $BF_3 \cdot Et_2O$ while cooling with an ice-MeOH bath. The mixture was agitated at the same temperature for 1 hour and at room temperature for 24 hours. The reaction mixture was filtered through Celite® and distilled under reduced pressure. The residue was purified by chromatography on silicagel (Wakogel® C—300, 20 g, toluene:ethyl acetate = 1:2). 20.9 mg of compound (26) were obtained (yield: 12.7%).

$R_f$ = 0.25 (toluene:ethyl acetate = 1:2).

$[\alpha]_D^{18}$ +8.80 (c = 0.25, $CHCl_3$).

Elemental analysis

| | | | |
|---|---|---|---|
| Calculated | C, 64.18%, | H, 8.64%, | N, 2.05 |
| Found | C, 64.03%, | H, 8.50%, | N, 1.80 |

Example 7

From compound (26) to compound (27)

15 mg (0.0989 mmol) of compound (26) were dissolved in a mixture of 1 ml of THF and 1 ml of MeOH. To the mixture were added 25 µl of 1N $NaOCH_3$ solution and the mixture was agitated overnight at room temperature. After the vacuum distillation of the reaction mixture, the residue obtained was mixed with 1 ml of THF, 1 ml of MeOH and 0.5 ml of water and agitated overnight at room temperature. The reaction

mixture was neutralized with Amberlite® IRC—50S, filtered and distilled under reduced pressure. The residue was purified by TLC (developed by $CHCl_3:MeOH:H_2O = 60:30:4.6$ and extracted by $CHCl_3:MeOH:H_2O = 5:5:1$) and Sephadex® LH—20 ($CHCl_3:MeOH:H_2O = 60:30:4.6$). 7.3 mg of compound (27) (yield: 67%) and 2.5 mg of compound (28) were obtained (yield: 23%).

Compound (28)
$R_f = 0.412$ ($CHCl_3:MeOH:H_2O = 60:30:4.6$)
$[\alpha]_D^{24}$ +15.60 (c = 0.125, $CHCl_3:MeOH = 2:1$).
NMR (400 MHz d-b DMSO, 65°C, TMS)
5.56, H-5', d.t, J = 15.1, 7.1;
5.37, H-4' d,d, J = 15.4, 6.6;
4.67 H-1aeq bs; 4.10, H-4a bs

Compound (27)
$R_f = 0.333$ ($CHCl_3:MeOH:H_2O = 60:30:4.6$)
$[\alpha]_D^{24}$ −13.34 (c = 0.365, $CHCl_3:MeOH = 2:1$).
NMR (400 MHz d-b DMSO, 65°C, TMS)
5.54, H-5', d,t, J = 1.54, 6.6;
5.39 H-4' d,d, J = 6.8, 15.6;
4.06 H-1aax d, J = 7b;
4.03 H-4a bs

**Claims for the Contracting States: BE CH DE FR GB IT LI/LU NL SE**

1. Sialosylcerebrosides represented by the general formula I

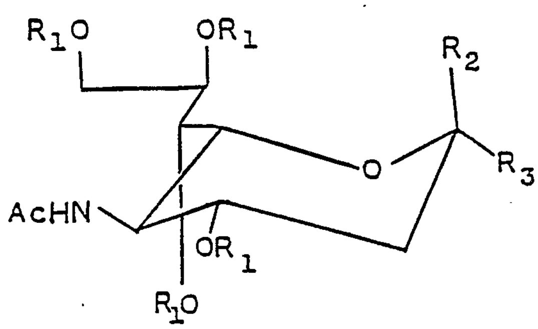

(I)

wherein $R_1$ is hydrogen or an acetyl group, $R_2$ is $—COOR_4$, wherein $R_4$ is hydrogen, sodium or a methyl group, and $R_3$ is the group

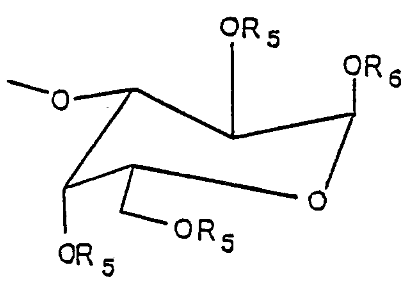

wherein $R_5$ is hydrogen or acetyl group, $R_6$ is $—C(CCl_3)=NH$ or the group

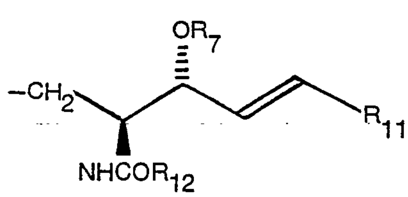

wherein $R_7$ is hydrogen or a benzoyl group, $R_{11}$ is a saturated aliphatic hydrocarbon group having 10 to 16

carbon atoms and $R_{12}$ is a saturated aliphatic hydrocarbon group having 15 to 25 carbon atoms, or $R_2$ is the group

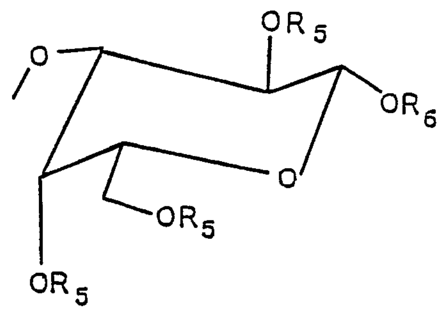

wherein $R_5$ and $R_6$ have the same meaning as defined above, and $R_3$ is —$COOR_4$, wherein $R_4$ has the same meaning as defined above.

2. Sialosylcerebrosides of claim 1, wherein $R_2$ is —$COOR_4$, wherein $R_4$ has the same meaning as defined above, and $R_3$ is the group

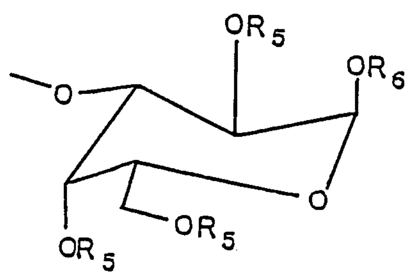

wherein $R_5$ and $R_6$ have the same meaning as defined above.

3. Sialosylcerebrosides of claim 2, wherein $R_1$ and $R_5$ are acetyl groups and $R_4$ is a methyl group.

4. Sialosylcerebrosides of claim 3, wherein $R_6$ is the group

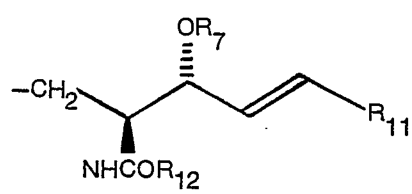

wherein $R_7$ is a benzoyl group and $R_{11}$ and $R_{12}$ have the same meaning as defined above.

5. Sialosylcerebrosides of claim 2, wherein $R_1$, $R_4$ and $R_5$ are hydrogen and $R_6$ is the group

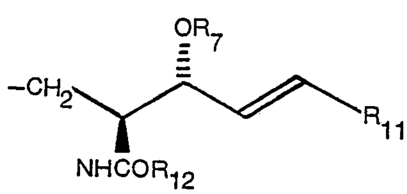

wherein $R_7$ is hydrogen and $R_{11}$ and $R_{12}$ have the same meaning as defined above.

6. Sialosylcerebrosides of claim 1, wherein $R_2$ is the group

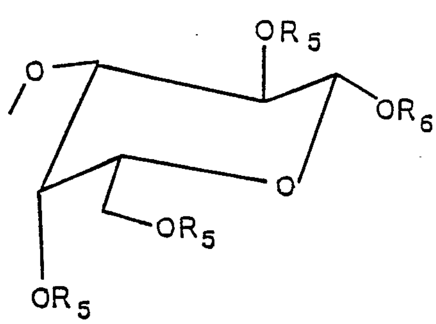

wherein $R_5$ and $R_6$ have the same meaning as defined above, and $R_3$ is $—COOR_4$, wherein $R_4$ has the same meaning as defined above.

7. Sialosylcerebrosides of claim 6, wherein $R_1$ and $R_5$ are acetyl groups and $R_4$ is a methyl group.

8. Sialosylcerebrosides of claim 7, wherein $R_6$ is the group

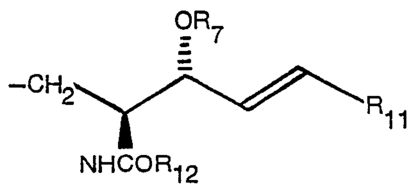

wherein $R_7$ is a benzoyl group and $R_{11}$ and $R_{12}$ have the same meaning as defined above.

9. Sialosylcerebrosides of claim 6, wherein $R_1$, $R_4$ and $R_5$ are hydrogen and $R_6$ is the group

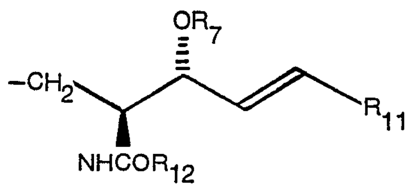

wherein $R_7$ is hydrogen and $R_{11}$ and $R_{12}$ have the same meaning as defined above.

10. Process for preparing sialosylcerebroside $GM_4$ analogues represented by the general formula III

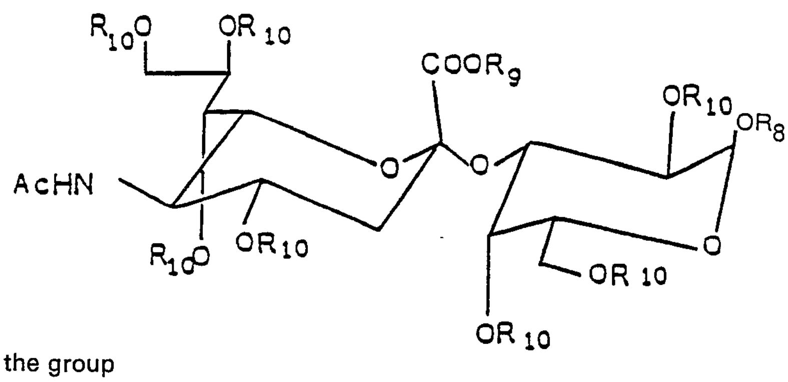

(III)

wherein $R_8$ is the group

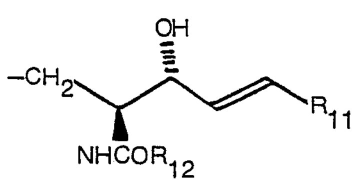

and $R_9$ and $R_{10}$ are hydrogen, $R_{11}$ is a saturated aliphatic hydrocarbon group having 10 to 16 carbon atoms and $R_{12}$ is a saturated aliphatic hydrocarbon group having 15 to 25 carbon atoms, comprising the steps of

(i) reacting a sialic acid derivative represented by the general formula II

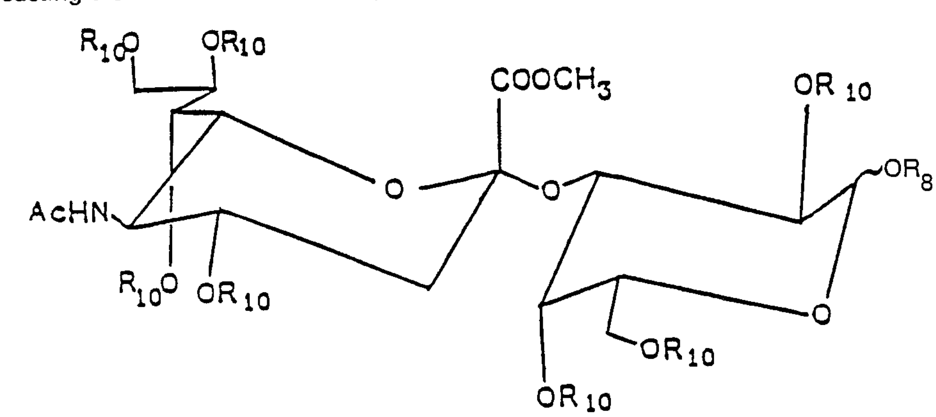

(II)

wherein $R_8$ is hydrogen and $R_{10}$ is an acetyl group, with trichloroacetonitrile to produce a sialic acid derivative represented by the general formula III, wherein $R_8$ is —$C(NH)CCl_3$ group, $R_9$ is methyl group and $R_{10}$ is an acetyl group,

(ii) reacting said sialic derivative with a benzoyl derivative of ceramide represented by the general formula

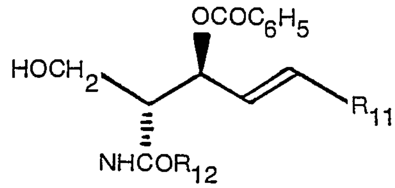

wherein $R_{11}$ and $R_{12}$ have the same meaning as defined above, to produce a benzoyl derivative of sialosylcerebroside represented by the general formula III, wherein $R_8$ is the group

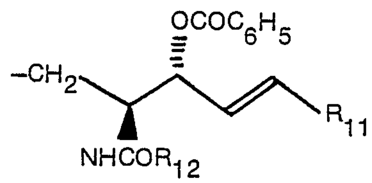

and $R_{11}$ and $R_{12}$ have the same meaning as defined above, $R_9$ is a methyl group and $R_{10}$ is an acetyl group, and

(iii) removing the acetyl and benzoyl group..

11. A process of claim 10, wherein the step (i) is carried out in dichloromethane in the presence of sodium hydride.

12. A process of claim 10, wherein the step (ii) is carried out in chloroform in the presence of molecular sieve 4A and $BF_3 \cdot Et_2O$.

13. A process of claim 10, wherein the step (iii) is carried out by treating the benzoyl derivative of sialosylcerebroside with NaOMe in a mixture of methanol and tetrahydrofuran.

14. Process for preparing sialosylcerebroside $GM_4$ analogues represented by the general formula III

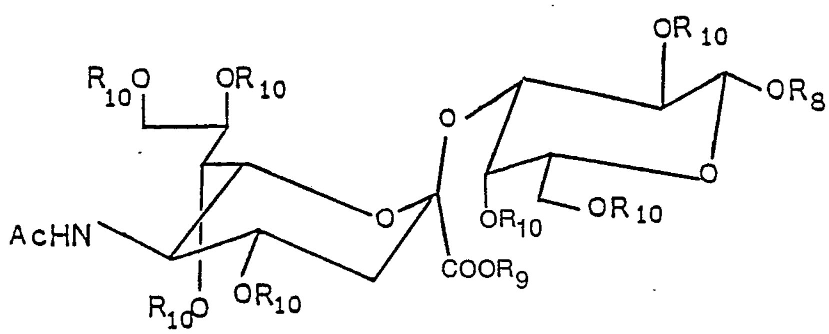

(III)

wherein $R_8$ is the group

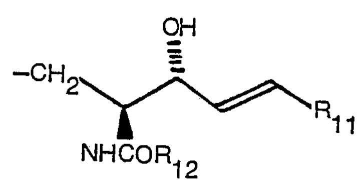

$R_{11}$ is a saturated aliphatic hydrocarbon group having 10 to 16 carbon atoms and $R_{12}$ is a saturated aliphatic hydrocarbon group having 15 to 25 carbon atoms and $R_9$ and $R_{10}$ are hydrogen, comprising the steps of

(i) reacting a sialic acid derivative represented by the general formula II

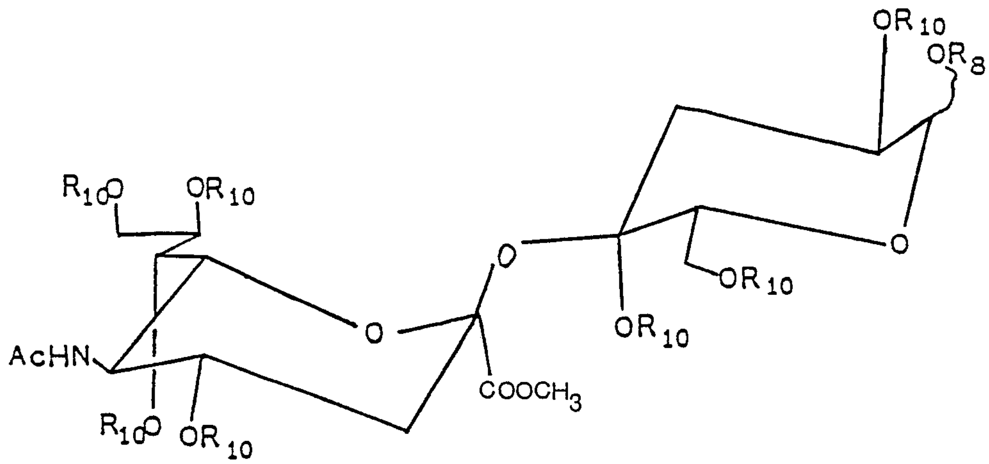

(II)

wherein $R_8$ is hydrogen and $R_{10}$ is an acetyl group, with trichloroacetonitrile to produce a sialic acid derivative represented by the general formula III, wherein $R_8$ is a $—C(NH)CCl_3$ group, $R_9$ is a methyl group and $R_{10}$ is an acetyl group,

(ii) reacting said sialic acid derivative with a benzoyl derivative of ceramide represented by the general formula

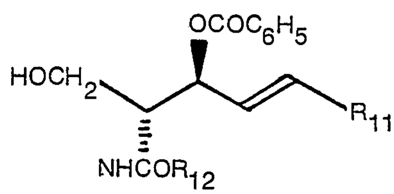

wherein $R_{11}$ and $R_{12}$ have the same meaning as defined above, to produce a benzoyl derivative of sialosylcerebroside represented by the general formula III, wherein $R_8$ is the group

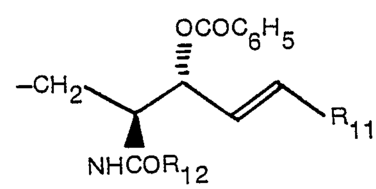

wherein $R_{11}$ and $R_{12}$ have the same meaning as defined above, $R_9$ is a methyl group and $R_{10}$ is an acetyl group, and

(iii) removing the acetyl and benzoyl group.

15. A process of claim 14 wherein the step (i) is carried out in dichloromethane in the presence of 1,8-diazabicyclo[5.4.0]undec-7-ene.

16. A process of claim 14 wherein the step (ii) is carried out in chloroform in the presence of molecular sieve 4A and $BF_3 \cdot Et_2O$.

17. A process of claim 14 wherein the step (iii) is carried out by treating the benzoyl derivative of sialosylcerebroside with NaOMe in a mixture of methanol and tetrahydrofuran.

**Claims for the Contracting State: AT**

1. Process for preparing sialosylcerebroside $GM_4$ analogues represented by the general formula III

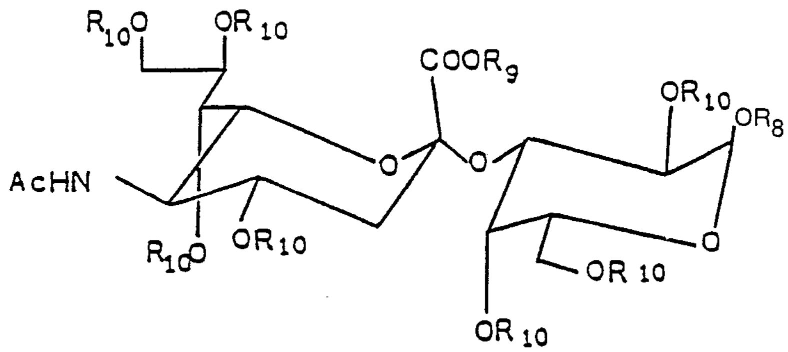

(III)

wherein $R_8$ is the group

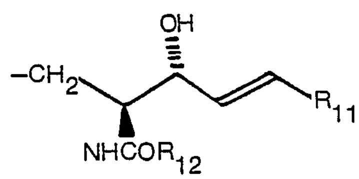

and $R_9$ and $R_{10}$ are hydrogen, $R_{11}$ is a saturated aliphatic hydrocarbon group having 10 to 16 carbon atoms and $R_{12}$ is a saturated aliphatic hydrocarbon group having 15 to 25 carbon atoms, comprising the steps of

(i) reacting a sialic acid derivative represented by the general formula II

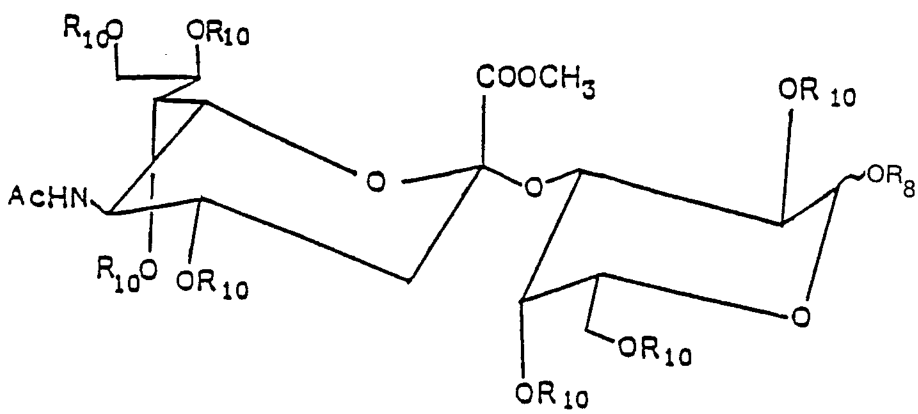

(II)

wherein $R_8$ is hydrogen and $R_{10}$ is an acetyl group, with trichloroacetonitrile to produce a sialic acid derivative represented by the general formula III, wherein $R_8$ is $-C(NH)CCl_3$ group, $R_9$ is methyl group and $R_{10}$ is an acetyl group,

(ii) reacting said sialic derivative with a benzoyl derivative of ceramide represented by the general formula

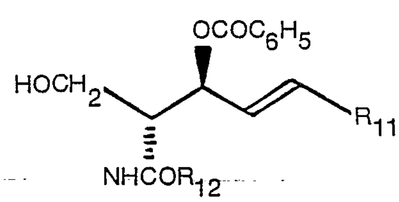

wherein $R_{11}$ and $R_{12}$ have the same meaning as defined above, to produce a benzoyl derivative of sialosylcerebroside represented by the general formula III, wherein $R_8$ is the group

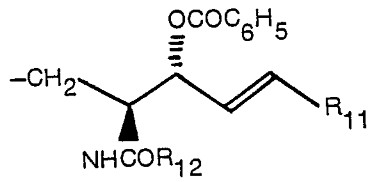

and $R_{11}$ and $R_{12}$ have the same meaning as defined above, $R_9$ is a methyl group and $R_{10}$ is an acetyl group, and

(iii) removing the acetyl and benzoyl group.

2. A process of claim 1, wherein the step (i) is carried out in dichloromethane in the presence of sodium hydride.

3. A process of claim 1, wherein the step (ii) is carried out in chloroform in the presence of molecular sieve 4A and $BF_3 \cdot Et_2O$.

4. A process of claim 1, wherein the step (iii) is carried out by treating the benzoyl derivative of sialosylcerebroside with NaOMe in a mixture of methanol and tetrahydrofuran.

5. Process for preparing sialosylcerebroside $GM_4$ analogues represented by the general formula III

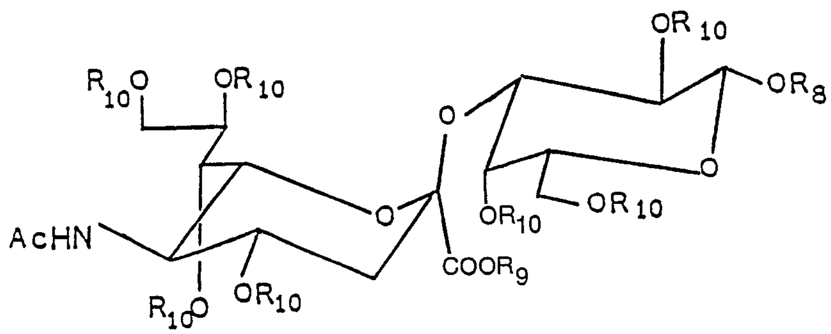

(III)

wherein $R_8$ is the group

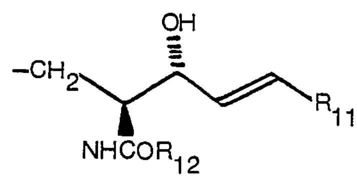

$R_{11}$ is a saturated aliphatic hydrocarbon group having 10 to 16 carbon atoms and $R_{12}$ is a saturated aliphatic hydrocarbon group having 15 to 25 carbon atoms and $R_9$ and $R_{10}$ are hydrogen, comprising the steps of

(i) reacting a sialic acid derivative represented by the general formula II

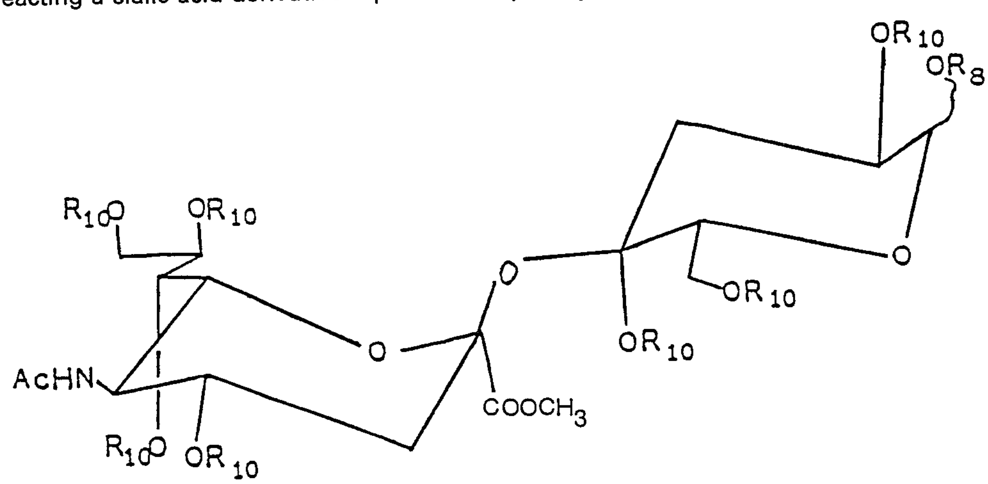

(II)

wherein $R_8$ is hydrogen and $R_{10}$ is an acetyl group, with trichloroacetonitrile to produce a sialic acid

derivative represented by the general formula III, wherein $R_8$ is a —C(NH)$CCl_3$ group, $R_9$ is a methyl group and $R_{10}$ is an acetyl group,

(ii) reacting said sialic acid derivative with a benzoyl derivative of ceramide represented by the general formula

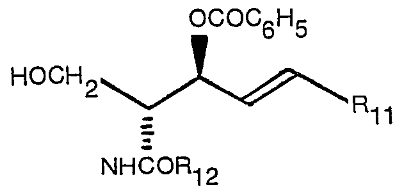

wherein $R_{11}$ and $R_{12}$ have the same meaning as defined above, to produce a benzoyl derivative of sialosylcerebroside represented by the general formula III, wherein $R_8$ is the group

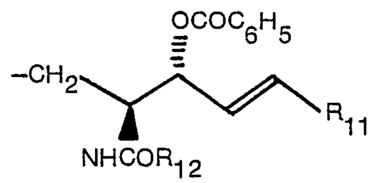

wherein $R_{11}$ and $R_{12}$ have the same meaning as defined above, $R_9$ is a methyl group and $R_{10}$ is an acetyl group, and

(iii) removing the acetyl and benzoyl group.

6. A process of claim 5 wherein the step (i) is carried out in dichloromethane in the presence of 1,8-diazabicyclo[5.4.0]undec-7-ene.

7. A process of claim 5 wherein the step (ii) is carried out in chloroform in the presence of molecular sieve 4A and $BF_3 \cdot Et_2O$.

8. A process of claim 5 wherein the step (iii) is carried out by treating the benzoyl derivative of sialosylcerebroside with NaOMe in a mixture of methanol and tetrahydrofuran.

**Patentansprüche für die Vertragsstaaten: BE CH DE FR GB IT LI/LU NL SE**

1. Sialosylcerebroside der allgemeinen Formel I,

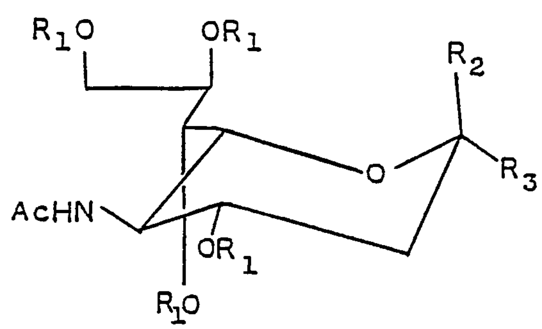

(I)

in der $R_1$ ein Wasserstoffatom oder eine Acetylgruppe ist, $R_2$ ein $COOR_4$-Rest ist, wobei $R_4$ ein Wasserstoff- oder Natriumatom oder eine Methylgruppe ist, und $R_3$ die Gruppe

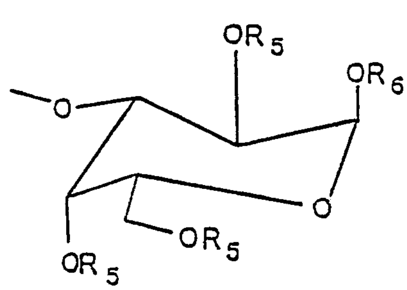

wobei $R_5$ ein Wasserstoffatom oder eine Acetylgruppe, $R_6$ eine $(CCl_3)(NH)C$-Gruppe oder die Gruppe

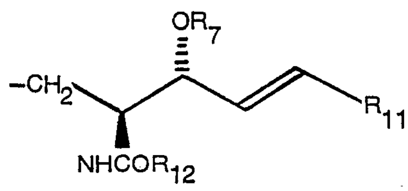

bedeutet, wobei $R_7$ Wasserstoffatom oder eine Benzoylgruppe, $R_{11}$ einen gesättigten aliphatischen Kohlenwasserstoffrest mit 10 bis 16 Kohlenstoffatomen und $R_{12}$ einen gesättigten aliphatischen Kohlenwasserstoffrest mit 15 bis 25 Kohlenstoffatomen darstellt, oder $R_2$ die Gruppe

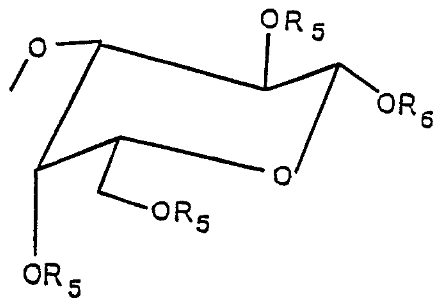

bedeutet, wobei $R_5$ und $R_6$ die vorstehend angegebene Bedeutung haben und $R_3$ ein $—COOR_4$-Rest ist, wobei $R_4$ die vorstehend angegebenen Bedeutung hat.

2. Sialosylcerebroside nach Anspruch 1, dadurch gekennzeichnet, daß $R_2$ ein $—COOR_4$-Rest ist, wobei $R_4$ die vorstehend angegebene Bedeutung hat, und $R_3$ die Gruppe

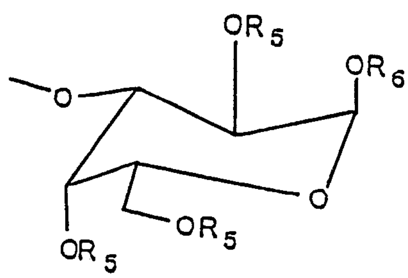

bedeutet, wobei $R_5$ und $R_6$ die vorstehend angegebene Bedeutung haben.

3. Sialosylcerebroside nach Anspruch 2, dadurch gekennzeichnet, daß $R_1$ und $R_5$ Acetylgruppen und $R_4$ eine Methylgruppe darstellen.

4. Sialosylcerebroside nach Anspruch 3, dadurch gekennzeichnet, daß $R_6$ die Gruppe

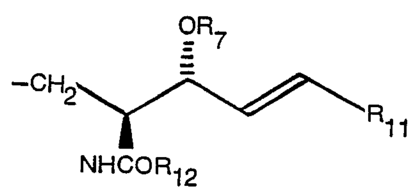

bedeutet, wobei $R_7$ eine Benzoylgruppe ist und $R_{11}$ und $R_{12}$ die vorstehend angegebene Bedeutung haben.

5. Sialosylcerebroside nach Anspruch 2, dadurch gekennzeichnet, daß $R_1$, $R_4$ und $R_5$ Wasserstoffatome sind, und $R_6$ die Gruppe

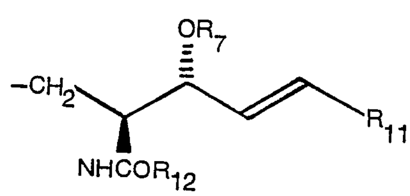

bedeutet, wobei $R_7$ ein Wasserstoffatom ist und $R_{11}$ und $R_{12}$ die vorstehend angegebene Bedeutung haben.

6. Sialosylcerebroside nach Anspruch 1, dadurch gekennzeichnet, daß $R_2$ die Gruppe

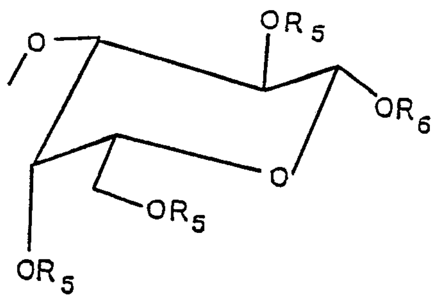

bedeutet, wobei $R_5$ und $R_6$ die vorstehend angegebene Bedeutung haben und $R_3$ ein $-COOR_4$-Rest ist, wobei $R_4$ die vorstehend angegebene Bedeutung hat.

7. Sialosylcerebroside nach Anspruch 6, dadurch gekennzeichnet, daß $R_1$ und $R_5$ Acetylgruppen und $R_4$ eine Methylgruppe darstellen.

8. Sialosylcerebroside nach Anspruch 7, dadurch gekennzeichnet, daß $R_6$ die Gruppe

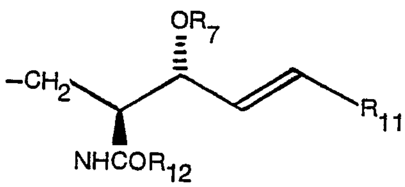

bedeutet, wobei $R_7$ eine Benzoylgruppe ist und $R_{11}$ und $R_{12}$ die vorstehend angegebene Bedeutung haben.

9. Sialosylcerebroside nach Anspruch 6, dadurch gekennzeichnet, daß $R_1$, $R_4$ und $R_5$ Wasserstoffatome sind, und $R_6$ die Gruppe

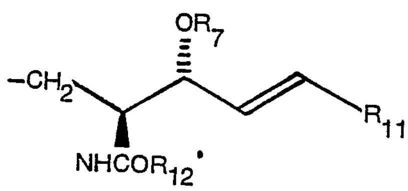

bedeutet, wobei $R_7$ ein Wasserstoffatom ist und $R_{11}$ und $R_{12}$ die vorstehend angegebene Bedeutung haben.

10. Verfahren zur Herstellung von Sialosylcerebrosid-$GM_4$-Analogen der allgemeinen Formel III

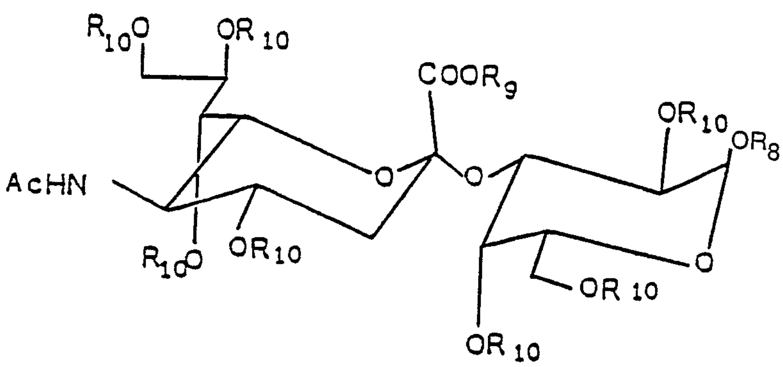

(III)

in der $R_8$ die Gruppe

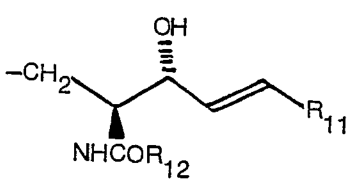

bedeutet, $R_9$ und $R_{10}$ Wasserstoffatome sind, $R_{11}$ ein gesättigter aliphatischer Kohlenwasserstoffrest mit 10 bis 16 Kohlenstoffatomen und $R_{12}$ ein gesättigter aliphatischer Kohlenwasserstoffrest mit 15 bis 25 Kohlenstoffatomen ist, das die Stufen umfaßt:

(i) Umsetzen eines Sialsäure-Derivats der allgemeinen Formel II

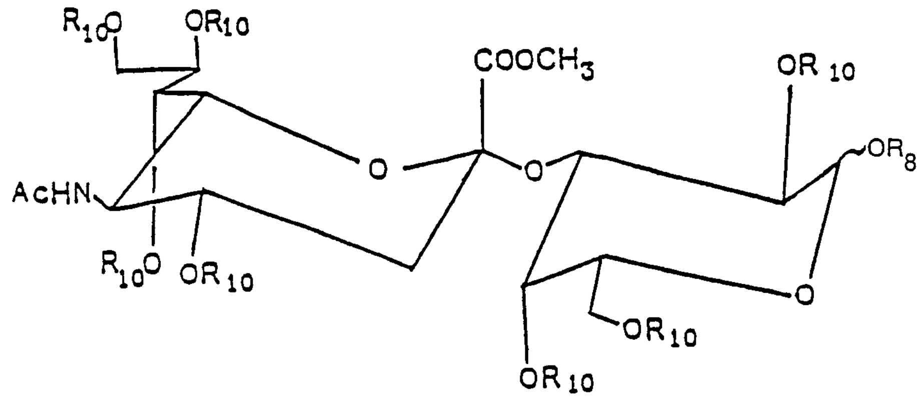

(II)

wobei $R_8$ ein Wasserstoffatom und $R_{10}$ eine Acetylgruppe ist, mit Trichloracetonitril zu einem Sialsäure-Derivat der allgemeinen Formel III, in der $R_8$ eine $CCl_3(NH)C$-Gruppe, $R_9$ eine Methylgruppe und $R_{10}$ eine Acetylgruppe ist,

(ii) Umsetzen dieses Sialsäure-Derivats mit einem Benzoyl-Derivat von Ceramid der nachstehend allgemeinen Formel

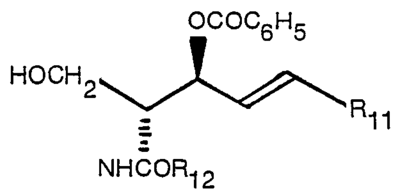

in der $R_{11}$ und $R_{12}$ die vorstehend angegebene Bedeutung haben, zu einem Benzoyl-Derivat von Sialosylcerebrosid der allgemeinen Formel III, in der $R_8$ die Gruppe

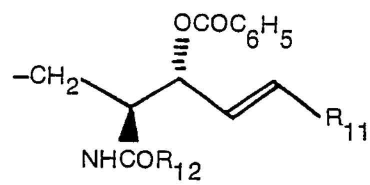

bedeutet, $R_{11}$ und $R_{12}$ die vorstehend angegebene Bedeutung haben, $R_9$ eine Methylgruppe und $R_{10}$ eine Acetylgruppe ist, und

(iii) Abspalten der Acetyl- und Benzoylgruppe.

11. Verfahren nach Anspruch 10, dadurch gekennzeichnet, daß die Stufe (1) in Dichlormethan in Gegenwart von Natriumhydrid durchgeführt wird.

12. Verfahren nach Anspruch 10, dadurch gekennzeichnet, daß die Stufe (2) in Chloroform in Gegenwart von 4 Å-Molekularsieb und $BF_3 \cdot Et_2O$ durchgeführt wird.

13. Verfahren nach Anspruch 10, dadurch gekennzeichnet, daß die Stufe (3) durch Umsetzen des Benzoyl-Derivats von Sialosylcerebrosid mit NaOME in einer Mischung aus Methanol und Tetrahydrofuran durchgeführt wird.

14. Verfahren zur Herstellung von Sialosylcerebrosid-$GM_4$-Analogen der allgemeinen Formel III

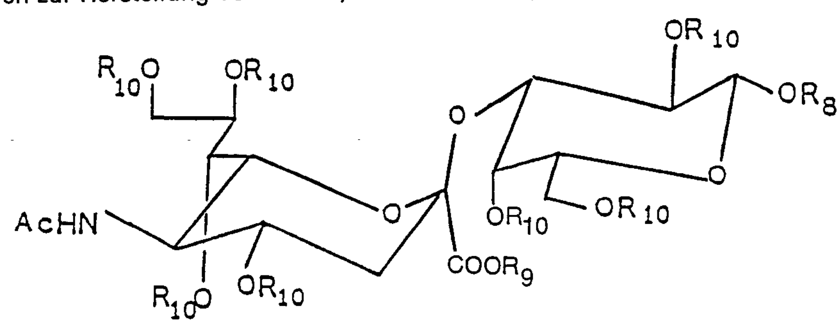

(III)

in der $R_8$ die Gruppe

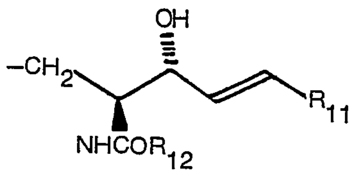

bedeutet, $R_9$ und $R_{10}$ Wasserstoffatome, $R_{11}$ ein gesättigter aliphatischer Kohlenwasserstoffrest mit 10 bis 16 Kohlenstoffatomen und $R_{12}$ ein gesättigter aliphatischer Kohlenwasserstoffrest mit 15 bis 25 Kohlenstoffatomen ist, das die Stufen umfaßt:

(i) Umsetzen eines Sialsäure-Derivats der allgemeinen Formel II

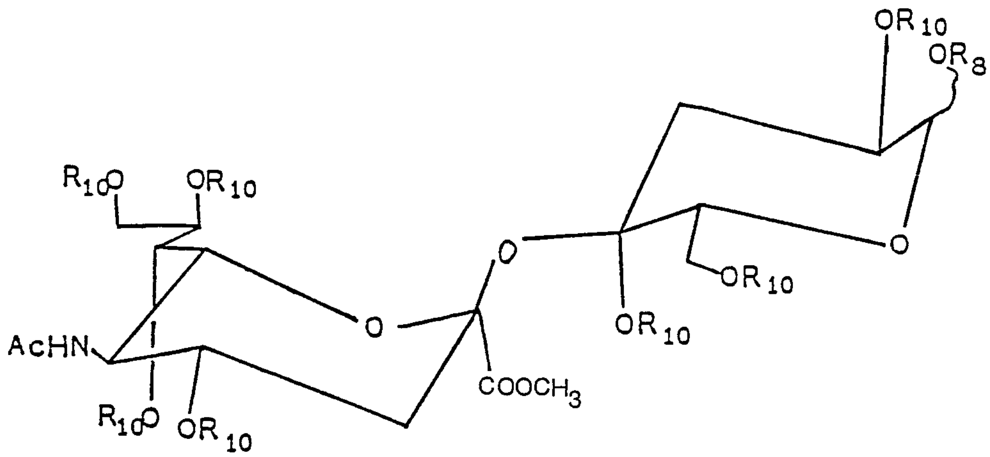

(II)

in der $R_8$ ein Wasserstoffatom und $R_{10}$ eine Acetylgruppe ist, mit Trichloracetonitril zu einem Sialsäure-Derivat der allgemeinen Formel III, in der $R_8$ eine $CCl_3(NH)C$-Gruppe, $R_9$ eine Methylgruppe und $R_{10}$ eine Acetylgruppe ist,

(ii) Umsetzen dieses Sialsäure-Derivats mit einem Benzoyl-Derivat von Ceramid der nachstehend allgemeinen Formel

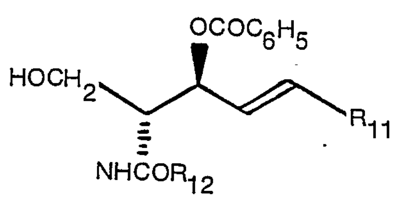

in der $R_{11}$ und $R_{12}$ die vorstehend angegebene Bedeutung haben, zu einem Benzoyl-Derivat von Sialosylcerebrosid der allgemeinen Formel III, in der $R_8$ die Gruppe

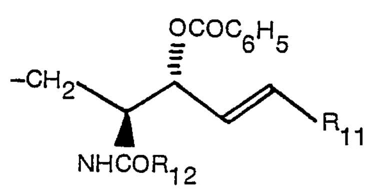

bedeutet, $R_{11}$ und $R_{12}$ die vorstehend angegebene Bedeutung haben, $R_9$ eine Methylgruppe und $R_{10}$ eine Acetylgruppe ist, und

(iii) Abspalten der Acetyl- und Benzoylgruppe.

15. Verfahren nach Anspruch 14, dadurch gekennzeichnet, daß die Stufe (1) in Dichlormethan in Gegenwart von 1,8-Diazabicyclo[5.4.0]undec-7-en durchgeführt wird.

16. Verfahren nach Anspruch 14, dadurch gekennzeichnet, daß die Stufe (2) in Chloroform in Gegenwart von 4 Å-Molekularsieb und $BF_3 \cdot Et_2O$ durchgeführt wird.

17. Verfahren nach Anspruch 14, dadurch gekennzeichnet, daß die Stufe (3) durch Umsetzen des Benzoyl-Derivats von Sialosylcerebrosid mit NaOMe in einer Mischung aus Methanol und Tetrahydrofuran durchgeführt wird.

**Patentansprüche für den Vertragsstaat: AT**

1. Verfahren zur Herstellung von Sialosylcerebrosid-$GM_4$-Analogen der allgemeinen Formel III

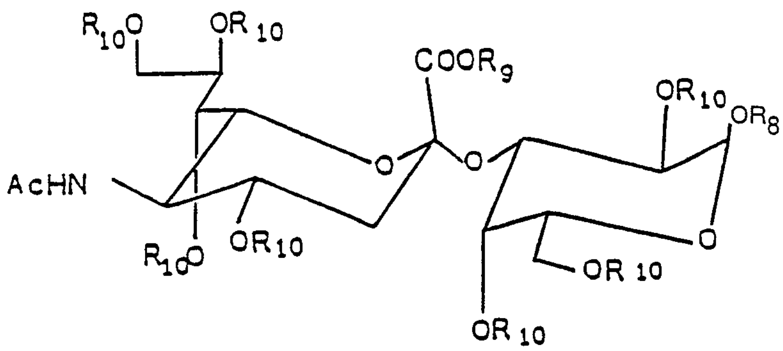

(III)

in der $R_8$ die Gruppe

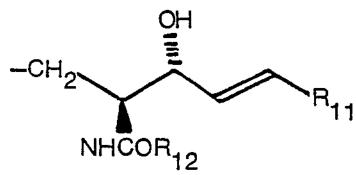

bedeutet, $R_9$ und $R_{10}$ Wasserstoffatome sind, $R_{11}$ ein gesättigter aliphatischer Kohlenwasserstoffrest mit 10 bis 16 Kohlenstoffatomen und $R_{12}$ ein gesättigter aliphatischer Kohlenwasserstoffrest mit 15 bis 25 Kohlenstoffatomen ist, das die Stufen umfaßt:

(i) Umsetzen eines Sialsäure-Derivats der allgemeinen Formel II

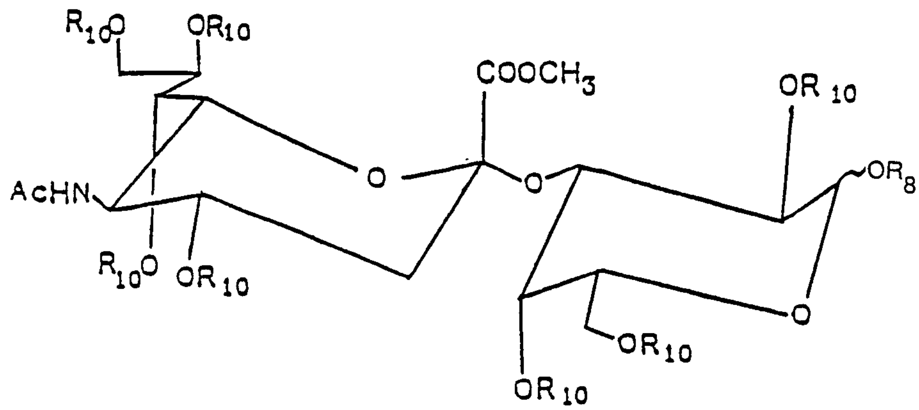

(II)

wobei $R_8$ ein Wasserstoffatom und $R_{10}$ eine Acetylgruppe ist, mit Trichloracetonitril zu einem Sialsäure-Derivat der allgemeinen Formel III, in der $R_8$ eine $CCl_3(NH)C$-Gruppe, $R_9$ eine Methylgruppe und $R_{10}$ eine Acetylgruppe ist,

(ii) Umsetzen dieses Sialsäure-Derivats mit einem Benzoyl-Derivat von Ceramid der nachstehend allgemeinen Formel

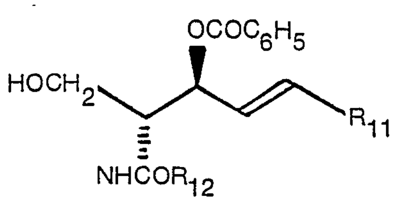

in der $R_{11}$ und $R_{12}$ die vorstehend angegebene Bedeutung haben, zu einem Benzoyl-Derivat von Sialosylcerebrosid der allgemeinen Formel III, in der $R_8$ die Gruppe

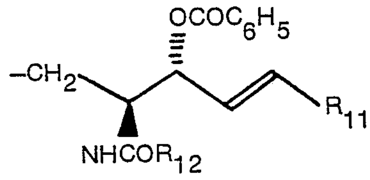

bedeutet, $R_{11}$ und $R_{12}$ die vorstehend angegebene Bedeutung haben, $R_9$ eine Methylgruppe und $R_{10}$ eine Acetylgruppe ist, und

(iii) Abspalten der Acetyl- und Benzoylgruppe.

2. Verfahren nach Anspruch 1, dadurch gekennzeichnet, daß die Stufe (1) in Dichlormethan in Gegenwart von Natriumhydrid durchgeführt wird.

3. Verfahren nach Anspruch 1, dadurch gekennzeichnet, daß die Stufe (2) in Chloroform in Gegenwart von 4 Å-Molekularsieb und $BF_3 \cdot Et_2O$ durchgeführt wird.

4. Verfahren nach Anspruch 1, dadurch gekennzeichnet, daß die Stufe (3) durch Umsetzen des Benzoyl-Derivats von Sialosylcerebrosid mit NaOMe in einer Mischung aus Methanol und Tetrahydrofuran durchgeführt wird.

5. Verfahren zur Herstellung von Sialosylcerebrosid-$GM_4$-Analogen der allgemeinen Formel III

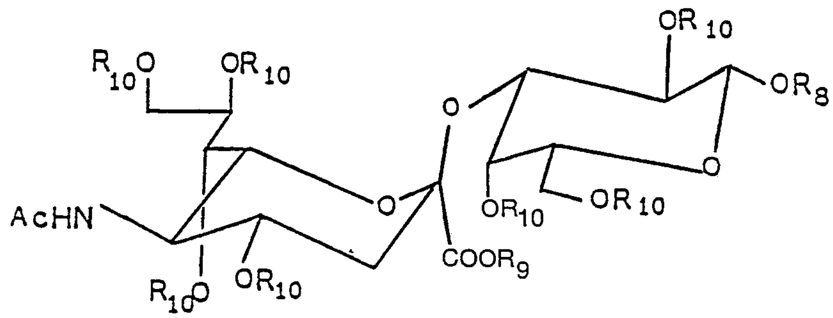

(III)

in der $R_8$ die Gruppe

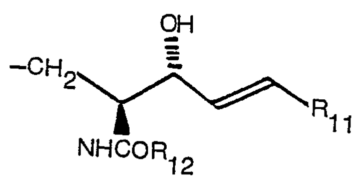

bedeutet, $R_9$ und $R_{10}$ Wasserstoffatome, $R_{11}$ ein gesättigter aliphatischer Kohlenwasserstoffrest mit 10 bis 16 Kohlenstoffatomen und $R_{12}$ ein gesättigter aliphatischer Kohlenwasserstoffrest mit 15 bis 25 Kohlenstoffatomen ist, das die Stufen umfaßt:

(i) Umsetzen eines Sialsäure-Derivats der allgemeinen Formel II

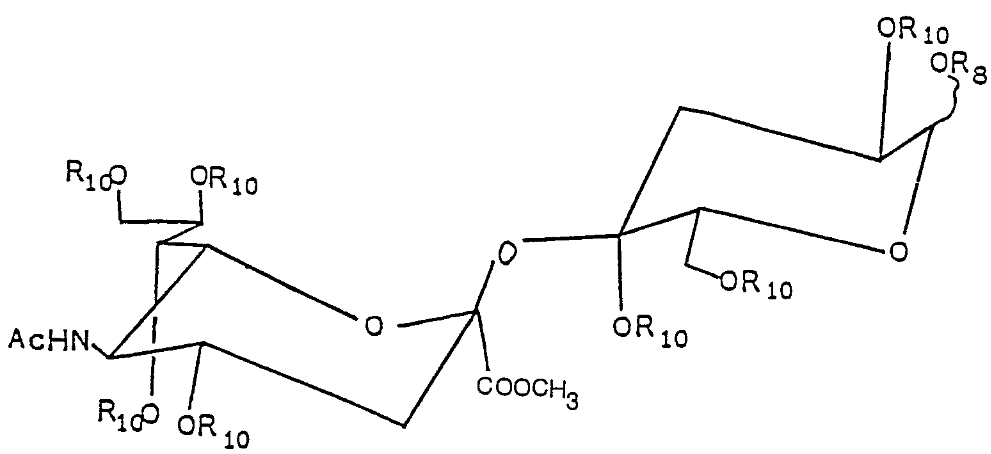

(II)

in der $R_8$ ein Wasserstoffatom und $R_{10}$ eine Acetylgruppe ist, mit Trichloracetonitril zu einem Sialsäure-Derivat der allgemeinen Formel III, in der $R_8$ eine $CCl_3(NH)C$-Gruppe, $R_9$ eine Methylgruppe und $R_{10}$ eine Acetylgruppe ist,

(ii) Umsetzen dieses Sialsäure-Derivats mit einem Benzoyl-Derivat von Ceramid der nachstehend allgemeinen Formel

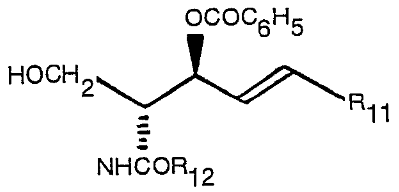

in der $R_{11}$ und $R_{12}$ die vorstehend angegebene Bedeutung haben, zu einem Benzoyl-Derivat von Sialosylcerebrosid der allgemeinen Formel III, in der $R_8$ die Gruppe

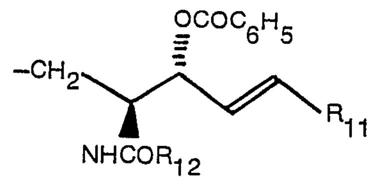

bedeutet, $R_{11}$ und $R_{12}$ die vorstehend angegebene Bedeutung haben, $R_9$ eine Methylgruppe und $R_{10}$ eine Acetylgruppe ist, und

(iii) Abspalten der Acetyl- und Benzoylgruppe.

6. Verfahren nach Anspruch 5, dadurch gekennzeichnet, daß die Stufe (1) in Dichlormethan in Gegenwart von Natriumhydrid durchgeführt wird.

7. Verfahren nach Anspruch 5, dadurch gekennzeichnet, daß die Stufe (2) in Chloroform in Gegenwart von 4 Å-Molekularsieb und $BF_3 \cdot Et_2O$ durchgeführt wird.

8. Verfahren nach Anspruch 5, dadurch gekennzeichnet, daß die Stufe (3) durch Umsetzen des Benzoyl-Derivats von Sialosylcerebrosid mit NaOME in einer Mischung aus Methanol und Tetrahydrofuran durchgeführt wird.

**Revendications pour les Etats contractants: BE CH DE FR GB IT LI LU NL SE**

1. Sialosylcérébrosides représentés par la formule générale I

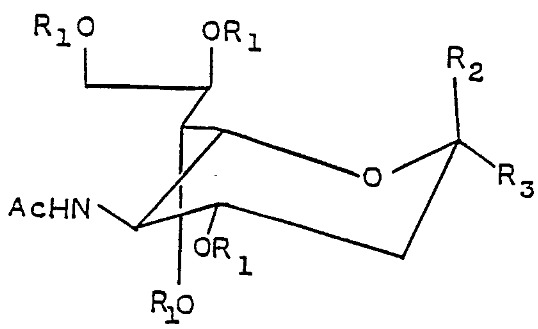

(I)

dans laquelle $R_1$ représente de l'hydrogène ou un groupe acétyle, $R_2$ représente —$COOR_4$, dans lequel $R_4$ représente de l'hydrogène, du sodium ou un groupe méthyle, et $R_3$ représente le groupe

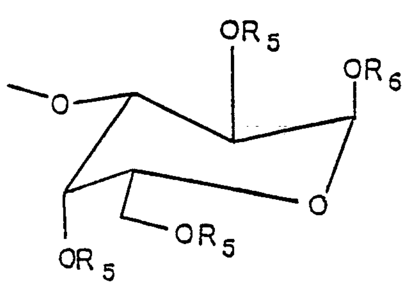

dans lequel $R_5$ représente de l'hydrogène ou un groupe acétyle, $R_6$ représente $-C(CCl_3)=NH$ ou le groupe

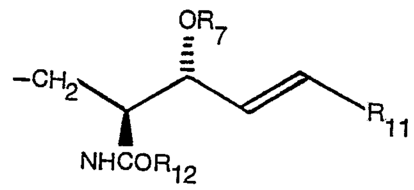

dans lequel $R_7$ représente de l'hydrogène ou un groupe benzoyle, $R_{11}$ représente un groupe hydrocarboné aliphatique saturé comportant 10 à 16 atomes de carbone et $R_{12}$ représente un groupe hydrocarboné aliphatique saturé comportant 15 à 25 atomes de carbone, ou $R_2$ représente le groupe

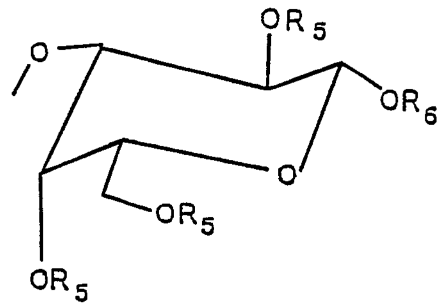

dans lequel $R_5$ et $R_6$ ont la même signification que donnée précédemment, et $R_3$ représente $-COOR_4$, dans lequel $R_4$ a la même signification que donnée précédemment.

2. Sialosylcérébrosides suivant la revendication 1, caractérisée en ce que $R_2$ représente $-COOR_4$, où $R_4$ a la même signification que donnée précédemment, et $R_3$ représente le groupe

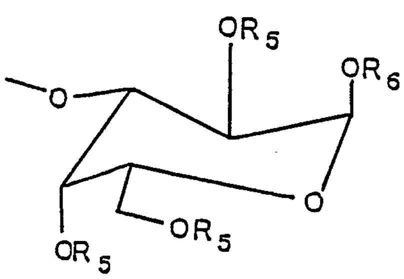

dans lequel $R_5$ et $R_6$ ont la même signification que donnée précédemment.

3. Sialosylcérébrosides suivant la revendication 2, caractérisés en ce que $R_1$ et $R_5$ représentent des groupes acétyle et $R_4$ représente un groupe méthyle.

4. Sialosylcérébrosides suivant la revendication 3, caractérisés en ce que $R_6$ représente le groupe

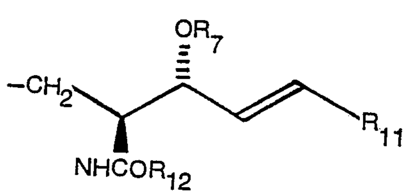

dans lequel $R_7$ représente un groupe benzoyle et $R_{11}$ et $R_{12}$ ont la même signification que donnée précédemment.

5. Sialosylcérébrosides suivant la revendication 2, caractérisés en ce que $R_1$, $R_4$ et $R_5$ représentent de l'hydrogène et $R_6$ représente le groupe

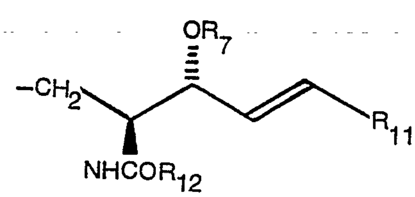

dans lequel $R_7$ représente de l'hydrogène et $R_{11}$ et $R_{12}$ ont la même signification que donnée précédemment.

6. Sialosylcérébrosides suivant la revendication 1, caractérisés en ce que $R_2$ représente le groupe

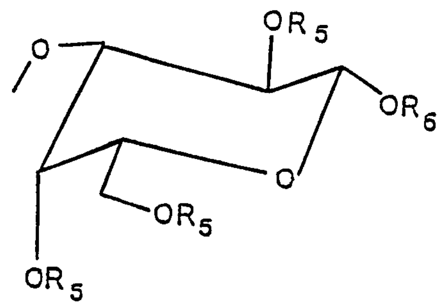

dans lequel $R_5$ et $R_6$ ont la même signification que donnée précédemment, et $R_3$ représente —$COOR_4$, dans lequel $R_4$ a la même signification que donnée précédemment.

7. Sialosylcérébrosides suivant la revendication 6, caractérisés en ce que $R_1$ et $R_5$ représentent des groupes acétyle et $R_4$ représente un groupe méthyle.

8. Sialosylcérébrosides suivant la revendication 7, caractérisés en ce que $R_6$ représente le groupe

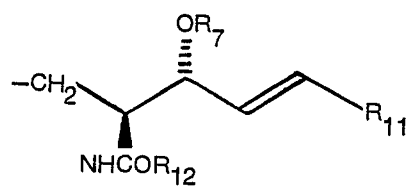

dans lequel $R_7$ représente un groupe benzoyle et $R_{11}$ et $R_{12}$ ont la mème significaiton que donnée précédemment.

9. Sialosylcérébrosides suivant la revendication 6, caractérisés en ce que $R_1$, $R_4$ et $R_5$ représentent de l'hydrogène et $R_6$ représente le groupe

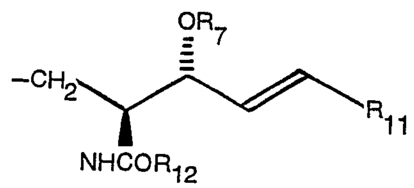

dans lequel $R_7$ représentee de l'hydrogène et $R_{11}$ et $R_{12}$ ont la mêmee signification que donnée précédemment.

10. Procédé de préparation d'analogues de silosylcérébroside $GM_4$ représentés par la formule générale III

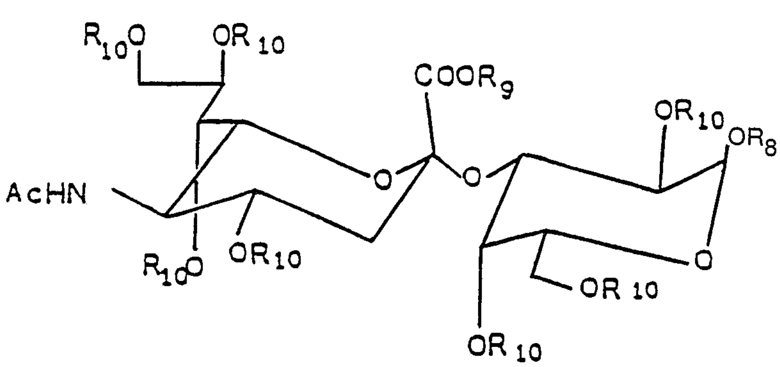

(III)

dans laquelle $R_8$ représente le groupe

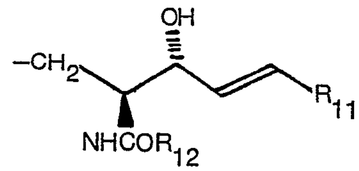

et $R_9$ et $R_{10}$ représentent de l'hydrogène, $R_{11}$ représente un groupe hydrocarboné aliphatique saturé comportant 10 à 16 atomes de carbone et $R_{12}$ représente un groupe hydrocarboné aliphatique saturé comportant 15 à 25 atomes de carbone, comprenant les étapes:

(i) de réaction d'un dérivé d'acide sialique représenté par la formule générale II

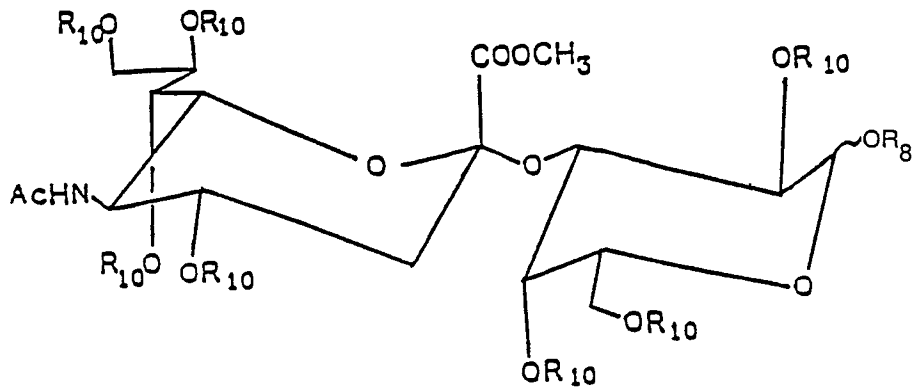

(II)

dans laquelle $R_8$ représente de l'hydrogène et $R_{10}$ représente un groupe acétyle, avec du trichloroacétonitrile pour produire un dérivé d'acide sialique représenté par la formule générale III, dans laquelle $R_8$ représente un groupe —$C(NH)]CCl_3$, $R_9$ représente un groupe méthyle et $R_{10}$ représente un groupe acétyle,

(ii) de réaction de ce dérivé sialique avec un dérivé benzoylé de céramide représenté par la formule générale

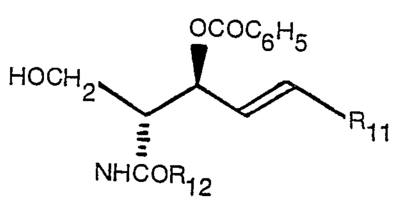

dans laquelle $R_{11}$ et $R_{12}$ ont la même signification que donnée précédemment, pour obtenir un dérivé benzoylé de sialosylcérébroside représenté par la formule générale III, dans laquelle $R_8$ représente le groupe

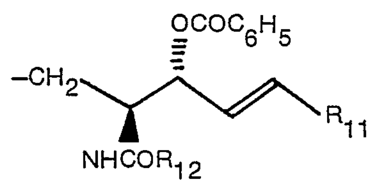

et $R_{11}$ et $R_{12}$ ont la même signification que donnée précédemment, $R_9$ est un groupe méthyle et $R_{10}$ est un groupe acétyle, et

(iii) de séparation du groupe acétyle et benzoyle.

11. Procédé suivant la revendication 10, caractérisé en ce que l'on réalise l'étape (i) dans du dichlorométhane en présence d'hydrure de sodium.

12. Procédé suivant la revendication 10, caractérisé en ce que l'on réalise l'étape (ii) dans du chloroforme en présence de tamis moléculaire 4A et de $BF_3 \cdot Et_2O$.

13. Procédé suivant la revendication 10, caractérisé en ce que l'on réalise l'étape (iii) par traitement du dérivé benzoylé de sialosylcérébroside par du NaOMe dans un mélange de méthanol et de tétrahydrofuranne.

14. Procédé de préparation d'analogues de sialosylcérébroside $GM_4$ représenté par la formule générale III

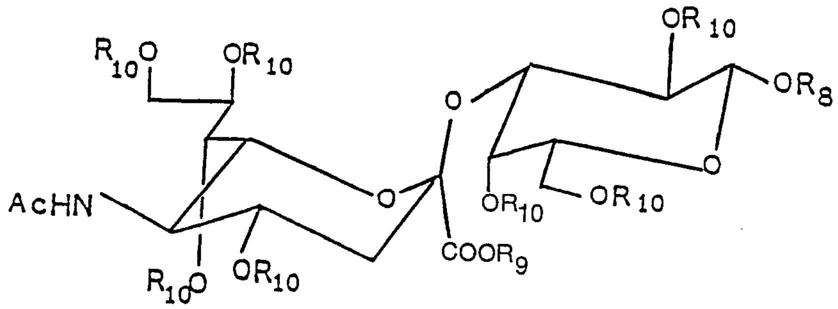

(III)

dans laquelle $R_8$ représente le groupe

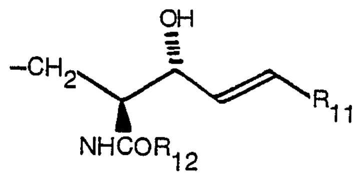

$R_{11}$ représente un groupe hydrocarboné aliphatique saturé comportant 10 à 16 atomes de carbone et $R_{12}$ représente un groupe hydrocarboné aliphatique saturé comportant 15 à 25 atomes de carbone et $R_9$ et $R_{10}$ représentent de l'hydrogène, comprenant les étapes:

(i) de réaction d'un dérivé d'acide sialique représenté par la formule générale II

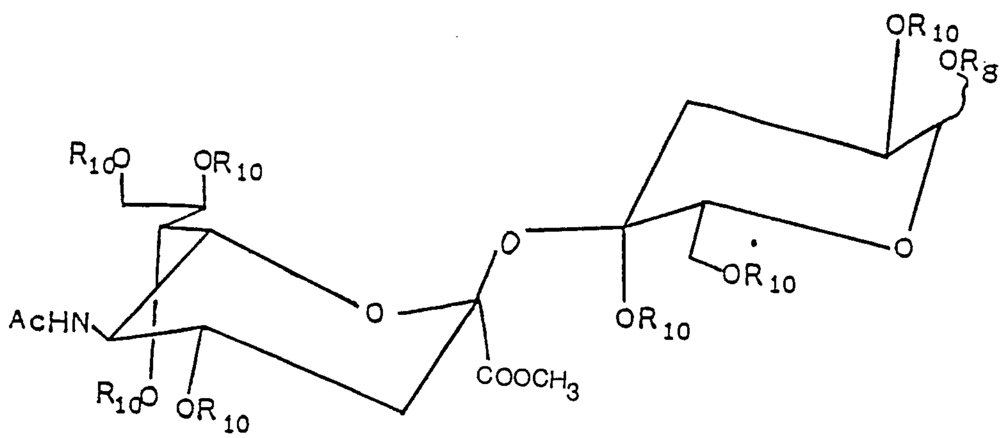

(II)

dans laquelle $R_8$ représente de l'hydrogène et $R_{10}$ représente un groupe acétyle, avec du trichloroacétonitrile pour produire un dérivé d'acide sialique représenté par la formule générale III, dans laquelle $R_8$ représente un groupe —C(NH)$CCl_3$, $R_9$ représente un groupe méthyle et $R_{10}$ représente un groupe acétyle,

(ii) de réaction de ce dérivé d'acide sialique avec un dérivé benzoyle de céramide représenté par la formule générale

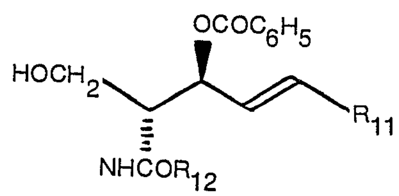

dans laquelle $R_{11}$ et $R_{12}$ ont la même signification qu donnée précédemment, pour obtenir un dérivé

benzoylé de sialosylcérébroside représenté par la formule générale III, dans laquelle $R_8$ représente le groupe

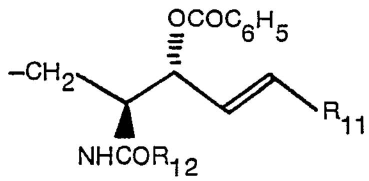

dans lequel $R_{11}$ et $R_{12}$ ont la même signification que donnée précédemment, $R_9$ représente un groupe méthyle et $R_{10}$ représente un groupe acétyle, et

(iii) de séparation du groupe acétyle et benzoyle.

15. Procédé suivant la revendication 14, caractérisé en ce que l'on réalise l'étape (i) dans du dichlorométhane en présence de 1,8-diazabicyclo[5.4.0]undéc-7-ène.

16. Procédé suivant la revendication 14, caractérisé en ce que l'on réalise l'étape (ii) dans du chloroforme en présence de tamis moléculaire 4A et de $BF_3 \cdot Et_2O$.

17. Procédé suivant la revendication 14, caractérisé en ce que l'on réalise l'étape (iii) par traitement du dérivé benzoylé de sialosylcérébroside par du NaOMe dans un mélange de méthanol et de tétrahydrofuranne.

**Revendications pour l'Etat contractant: AT**

1. Procédé de préparation d'analogues de sialosylcérébroside $GM_4$ représentés par la formule générale III

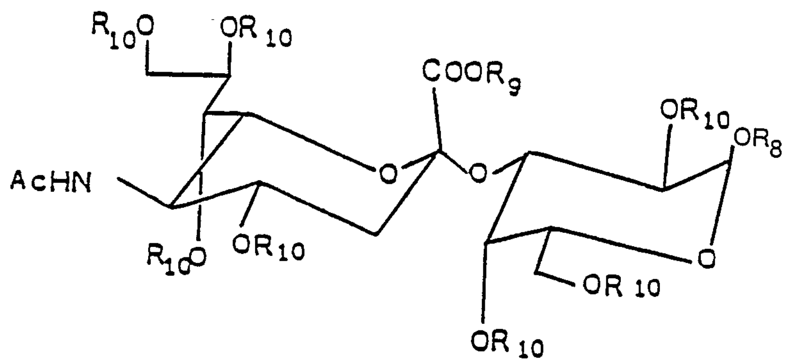

(III)

dans laquelle $R_8$ représente le groupe

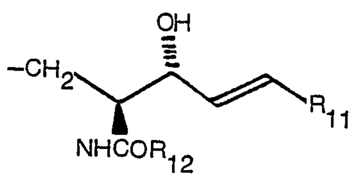

et $R_9$ et $R_{10}$ représentent de l'hydrogène, $R_{11}$ représente un groupe hydrocarboné aliphatique saturé comportant 10 à 16 atomes de carbone et $R_{12}$ représente un groupe hydrocarboné aliphatique saturé comportant 15 à 25 atomes de carbone, comprenant les étapes:

(i) de réaction d'un dérivé d'acide sialique représenté par la formule générale II

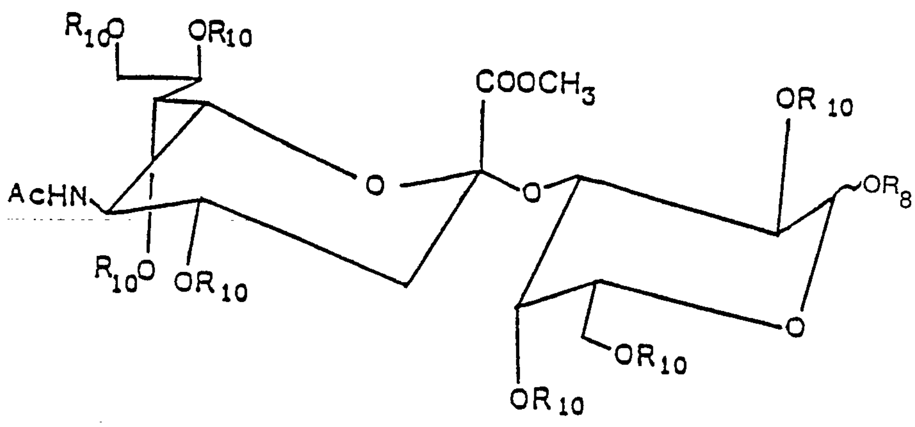

(II)

dans laquelle $R_8$ représente de l'hydrogène et $R_{10}$ représente un groupe acétyle, avec du trichloroacétonitrile pour produire un dérivé d'acide sialique représenté par la formule générale III, dans laquelle $R_8$ représente un groupe —$C(NH)CCl_3$, $R_9$ représente un groupe méthyle et $R_{10}$ représente un groupe acétyle,

(ii) de réaction de ce dérivé d'acide sialique avec un dérivé benzoyle de céramide représenté par la formule générale

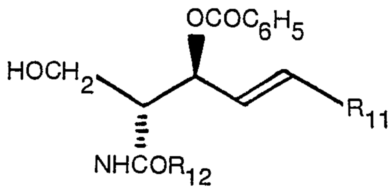

dans laquelle $R_{11}$ et $R_{12}$ ont la même signification qu donnée précédemment, pour obtenir un dérivé benzoylé de sialosylcérébroside représenté par la formule générale III, dans laquelle $R_8$ représente le groupe

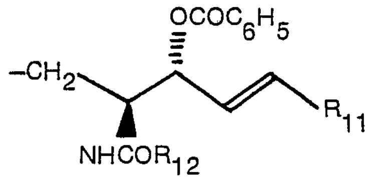

et $R_{11}$ et $R_{12}$ ont la même signification que donnée précédemment, $R_9$ représente un groupe méthyle et $R_{10}$ est un groupe acétyle, et

(iii) de séparation du groupe acétyle et benzoyle.

2. Procédé suivant la revendication 1, caractérisé en ce que l'on réalise l'étape (i) dans du dichlorométhane en présence d'hydrure de sodium.

3. Procédé suivant la revendication 1, caractérisé en ce que l'on réalise l'étape (ii) dans du chloroforme en présence de tamis moléculaire 4A et de $BF_3 \cdot Et_2O$.

4. Procédé suivant la revendication 1, caractérisé en ce que l'on réalise l'étape (iii) par traitement du dérivé benzoylé de sialosylcérébroside par du NaOMe dans un mélange de méthanol et de tétrahydrofuranne.

5. Procédé de préparation d'analogues de silosylcérébroside $GM_4$ représentés par la formule générale III

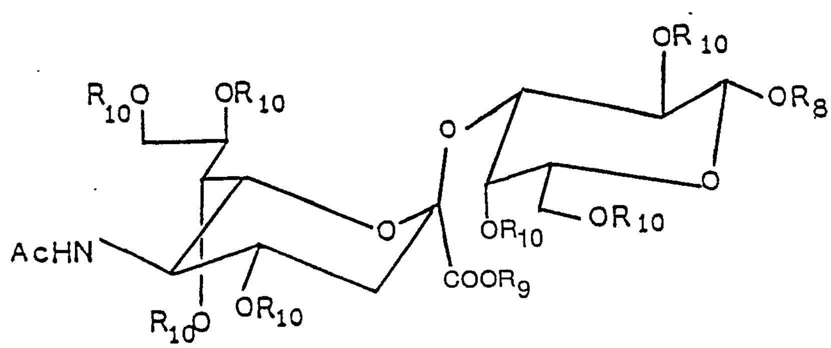

(III)

dans laquelle $R_8$ représente le groupe

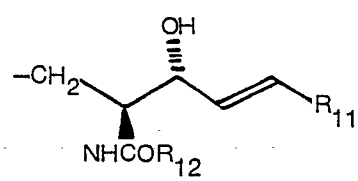

$R_{11}$ représente un groupe hydrocarboné aliphatique saturé comportant 10 à 16 atomes de carbone et $R_{12}$ représente un groupe hydrocarboné aliphatique saturé comportant 15 à 25 atomes de carbone et $R_9$ et $R_{10}$ représentent de l'hydrogène, comprenant les étapes:

(i) de réaction d'un dérivé d'acide sialique représenté par la formule générale II

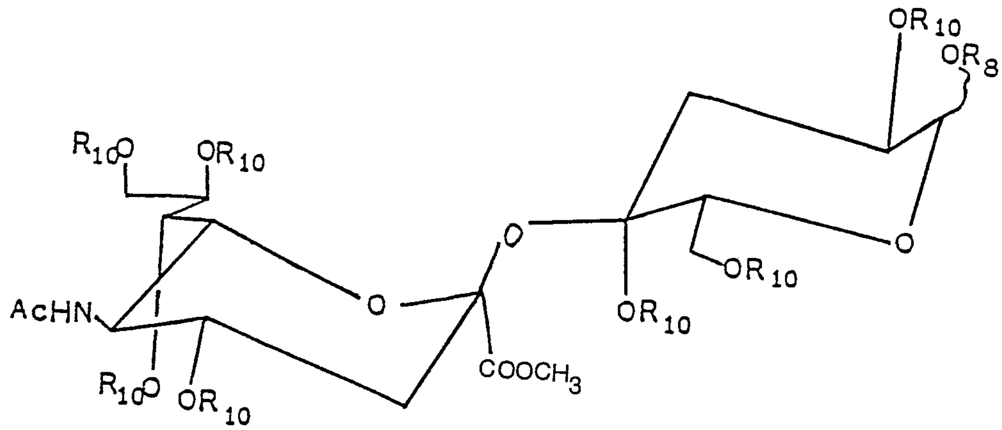

(II)

dans laquelle $R_8$ représente de l'hydrogène et $R_{10}$ représente un groupe acétyle, avec du trichloroacétonitrile pour produire un dérivé d'acide sialique représenté par la formule générale III, dans laquelle $R_8$ représente un groupe $-C(NH)CCl_3$, $R_9$ représente un groupe méthyle et $R_{10}$ représente un groupe acétyle,

(ii) de réaction de ce dérivé sialique avec un dérivé benzoylé de céramide représenté par la formule générale

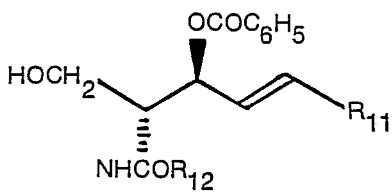

dans laquelle $R_{11}$ et $R_{12}$ ont la même signification que donnée précédemment, pour obtenir un dérivé benzoylé de sialosylcérébroside représenté par la formule générale III, dans laquelle $R_8$ représente le groupe

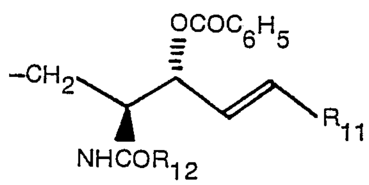

dans lequel $R_{11}$ et $R_{12}$ ont la même signification que donnée précédemment, $R_9$ représente un groupe méthyle et $R_{10}$ est un groupe acétyle, et

(iii) de séparation du groupe acétyle et benzoyle.

6. Procédé suivant la revendication 5, caractérisé en ce que l'on réalise l'étape (i) dans du dichlorométhane en présence de 1,8-diazabicyclo[5.4.0]undéc-7-ène.

7. Procédé suivant la revendication 5, caractérisé en ce que l'on réalise l'étape (ii) dans du chloroforme en présence de tamis moléculaire 4A et de $BF_3 \cdot Et_2O$.

8. Procédé suivant la revendication 5, caractérisé en ce que l'on réalise l'étape (iii) par traitement du dérivé benzoylé de sialosylcérébroside par du NaOMe dans un mélange de méthanol et de tétrahydrofuranne.